# EUROPEAN PATENT APPLICATION

(11) **EP 2 762 150 A1**
(43) Date of publication of application: **06.08.2014**
(21) Application number: 14159411.9
(22) Date of filing: 10.03.2010
(51) Int. Cl.: A61K 38/23, A61K 9/00, A61P 3/10, A61P 5/50, A61K 47/18

(54) **Treatment of Diabetes and Metabolic Syndrome**

(30) Priority: 12.03.2009 GB 0904271; 24.06.2009 GB 0910904; 24.07.2009 GB 0912906
(62) Divisional of application: 10708193.7
(71) Applicant: Nordic Bioscience A/S, 2730 Herlev (DK)
(72) Inventor: Karsdal, Morten Asser, DK-2100 Copenhagen (DK); Christiansen, Claus, CH-6922 Morcote (CH)
(74) Representative: Beck Greener

(57) **Abstract**

Enterally administered calcitonin family members other than amylin, particularly calcitonin itself, are effective to treat Type I diabetes, Type II diabetes or metabolic syndrome, for mitigating insulin resistance, and for reducing serum glucose levels.

SEQUENCE LISTING

<110>  Nordic Bioscience A/S

<120>  Treatment of Diabetes and Metabolic Syndrome

<130>  PJS/P16582WO

<150>  GB 0904271.4
<151>  2009-03-12

<150>  GB 0910904.2
<151>  2009-06-24

<150>  GB 0912906.5
<151>  2009-07-24

<160>  56

<170>  PatentIn version 3.3

<210>  1
<211>  32
<212>  PRT
<213>  Homo sapiens

<400>  1

Cys Gly Asn Leu Ser Thr Cys Met Leu Gly Thr Tyr Thr Gln Asp Phe
1               5                   10                  15

Asn Lys Phe His Thr Phe Pro Gln Thr Ala Ile Gly Val Gly Ala Pro
            20                  25                  30

<210>  2
<211>  32
<212>  PRT
<213>  Salmon

<400>  2

Cys Ser Asn Leu Ser Thr Cys Val Leu Gly Lys Leu Ser Gln Glu Leu
1               5                   10                  15

His Lys Leu Gln Thr Tyr Pro Arg Thr Asn Thr Gly Ser Gly Thr Pro
            20                  25                  30

<210>  3
<211>  32
<212>  PRT
<213>  Mus musculus

<400>  3

Cys Gly Asn Leu Ser Thr Cys Met Leu Gly Thr Tyr Thr Gln Asp Leu
1               5                   10                  15

Asn Lys Phe His Thr Phe Pro Gln Thr Ser Ile Gly Val Glu Ala Pro

20                  25                  30

<210>  4
<211>  32
<212>  PRT
<213>  Gallus gallus domesticus

<400>  4

Cys Ala Ser Leu Ser Thr Cys Val Leu Gly Lys Leu Ser Gln Glu Leu
1               5                   10                  15

His Lys Leu Gln Thr Tyr Pro Arg Thr Asp Val Gly Ala Gly Thr Pro
            20                  25                  30

<210>  5
<211>  32
<212>  PRT
<213>  Anguilla anguilla

<400>  5

Cys Ser Asn Leu Ser Thr Cys Val Leu Gly Lys Leu Ser Gln Glu Leu
1               5                   10                  15

His Lys Leu Gln Thr Tyr Pro Arg Thr Asp Val Gly Ala Gly Thr Pro
            20                  25                  30

<210>  6
<211>  32
<212>  PRT
<213>  Rattus

<400>  6

Cys Gly Asn Leu Ser Thr Cys Met Leu Gly Thr Tyr Thr Gln Asp Leu
1               5                   10                  15

Asn Lys Phe His Thr Phe Pro Gln Thr Ser Ile Gly Val Gly Ala Pro
            20                  25                  30

<210>  7
<211>  32
<212>  PRT
<213>  Equus ferus caballus

<400>  7

Cys Ser Asn Leu Ser Thr Cys Val Leu Gly Thr Tyr Thr Gln Asp Leu
1               5                   10                  15

Asn Lys Phe His Thr Phe Pro Gln Thr Ala Ile Gly Val Gly Ala Pro
            20                  25                  30

<210>  8
<211>  32
<212>  PRT
<213>  Canis lupus familiaris

<400>  8

Cys Ser Asn Leu Ser Thr Cys Val Leu Gly Thr Tyr Ser Lys Asp Leu
1               5                   10                  15

Asn Asn Phe His Thr Phe Ser Gly Ile Gly Phe Gly Ala Glu Thr Pro
            20                  25                  30

<210>  9
<211>  32
<212>  PRT
<213>  Canis lupus familiaris

<400>  9

Cys Ser Asn Leu Ser Thr Cys Val Leu Gly Thr Tyr Thr Gln Asp Leu
1               5                   10                  15

Asn Lys Phe His Thr Phe Pro Gln Thr Ala Ile Gly Val Gly Ala Pro
            20                  25                  30

<210>  10
<211>  32
<212>  PRT
<213>  Sus domestica

<400>  10

Cys Ser Asn Leu Ser Thr Cys Val Leu Ser Ala Tyr Trp Arg Asn Leu
1               5                   10                  15

Asn Asn Phe His Arg Phe Ser Gly Met Gly Phe Gly Pro Glu Thr Pro
            20                  25                  30

<210>  11
<211>  32
<212>  PRT
<213>  Artificial

<220>
<223>  General formula

<220>
<221>  MISC_FEATURE
<222>  (2)..(2)
<223>  A, G, OR S

<220>
<221>  MISC_FEATURE
<222>  (3)..(3)
<223>  N OR S

<220>
<221>  MISC_FEATURE
<222>  (8)..(8)
<223>  M OR V

<220>
<221>  MISC_FEATURE
<222>  (10)..(10)
<223>  G OR S

<220>
<221>  MISC_FEATURE
<222>  (11)..(11)
<223>  T, K OR A

<220>
<221>  MISC_FEATURE
<222>  (12)..(12)
<223>  L OR Y

<220>
<221>  MISC_FEATURE
<222>  (13)..(13)
<223>  T, S OR W

<220>
<221>  MISC_FEATURE
<222>  (14)..(14)
<223>  Q, K, OR R

<220>
<221>  MISC_FEATURE
<222>  (15)..(15)
<223>  D, E, OR N

<220>
<221>  MISC_FEATURE
<222>  (16)..(16)
<223>  F OR L

<220>
<221>  MISC_FEATURE
<222>  (17)..(17)
<223>  N OR H

<220>
<221>  MISC_FEATURE
<222>  (18)..(18)
<223>  K OR N

<220>
<221>  MISC_FEATURE
<222>  (19)..(19)
<223>  F OR L

<220>
<221>  misc_feature
<222>  (20)..(20)
<223>  Xaa can be any naturally occurring amino acid

<220>
<221>  MISC_FEATURE

<222>  (21)..(21)
<223>  T OR R

<220>
<221>  MISC_FEATURE
<222>  (22)..(22)
<223>  F OR Y

<220>
<221>  MISC_FEATURE
<222>  (23)..(23)
<223>  P OR S

<220>
<221>  MISC_FEATURE
<222>  (24)..(24)
<223>  Q, G, OR R

<220>
<221>  MISC_FEATURE
<222>  (25)..(25)
<223>  T, I, OR M

<220>
<221>  MISC_FEATURE
<222>  (26)..(26)
<223>  A, N, D, S, OR G

<220>
<221>  MISC_FEATURE
<222>  (27)..(27)
<223>  I, T, V, OR F

<220>
<221>  MISC_FEATURE
<222>  (29)..(29)
<223>  V, S, A, OR P

<220>
<221>  misc_feature
<222>  (30)..(30)
<223>  Xaa can be any naturally occurring amino acid

<220>
<221>  MISC_FEATURE
<222>  (31)..(31)
<223>  G OR E

<220>
<221>  MISC_FEATURE
<222>  (32)..(32)
<223>  A OR T

<400>  11

Cys Xaa Xaa Leu Ser Thr Cys Xaa Leu Xaa Xaa Xaa Xaa Xaa Xaa Xaa
1               5                   10                  15

Xaa Xaa Xaa Xaa Xaa Xaa Xaa Xaa Xaa Xaa Xaa Gly Xaa Xaa Xaa Pro
            20                  25                  30

<210>  12
<211>  32
<212>  PRT
<213>  Artificial

<220>
<223>  General formula


<220>
<221>  MISC_FEATURE
<222>  (11)..(11)
<223>  T OR K

<220>
<221>  MISC_FEATURE
<222>  (13)..(13)
<223>  T OR S

<220>
<221>  MISC_FEATURE
<222>  (15)..(15)
<223>  D OR E

<220>
<221>  MISC_FEATURE
<222>  (24)..(24)
<223>  Q OR R

<220>
<221>  MISC_FEATURE
<222>  (29)..(29)
<223>  V, S, OR A

<400>  12

Cys Ser Asn Leu Ser Thr Cys Val Leu Gly Xaa Leu Xaa Gln Xaa Leu
1               5                   10                  15


His Lys Leu Gln Thr Tyr Pro Xaa Thr Asn Thr Gly Xaa Gly Thr Pro
            20                  25                  30


<210>  13
<211>  37
<212>  PRT
<213>  Homo sapiens

<400>  13

<210>  14
<211>  37
<212>  PRT
<213>  Mus musculus

<400>  14

Lys Cys Asn Thr Ala Thr Cys Ala Thr Gln Arg Leu Ala Asn Phe Leu
1               5                   10                  15

Val Arg Ser Ser Asn Asn Leu Gly Pro Val Leu Pro Pro Thr Asn Val
            20                  25                  30

Gly Ser Asn Thr Tyr
        35

<210>  15
<211>  37
<212>  PRT
<213>  Homo sapiens

<400>  15

Ala Cys Asp Thr Ala Thr Cys Val Thr His Arg Leu Ala Gly Leu Leu
1               5                   10                  15

Ser Arg Ser Gly Gly Val Val Lys Asn Asn Phe Val Pro Thr Asn Val
            20                  25                  30

Gly Ser Lys Ala Phe
        35

<210>  16
<211>  37
<212>  PRT
<213>  Sus domestica

<400>  16

Ser Cys Asn Thr Ala Thr Cys Val Thr His Arg Leu Ala Gly Leu Leu
1               5                   10                  15

Ser Arg Ser Gly Gly Met Val Lys Ser Asn Phe Val Pro Thr Asp Val
            20                  25                  30

Gly Ser Glu Ala Phe
        35

<210>  17
<211>  47
<212>  PRT
<213>  Homo sapiens

<400>  17

Thr Gln Ala Gln Leu Leu Arg Val Gly Cys Val Leu Gly Thr Cys Gln
1               5                   10                  15

Val Gln Asn Leu Ser His Arg Leu Trp Gln Leu Met Gly Pro Ala Gly
            20                  25                  30

Arg Gln Asp Ser Ala Pro Val Asp Pro Ser Ser Pro His Ser Tyr
        35                  40                  45

<210>  18
<211>  52
<212>  PRT
<213>  Sus domestica

<400>  18

Tyr Arg Gln Ser Met Asn Asn Phe Gln Gly Leu Arg Ser Phe Gly Cys
1               5                   10                  15

Arg Phe Gly Thr Cys Thr Val Gln Lys Leu Ala His Gln Ile Tyr Gln
            20                  25                  30

Phe Thr Asp Lys Asp Lys Asp Gly Val Ala Pro Arg Ser Lys Ile Ser
        35                  40                  45

Pro Gln Gly Tyr
    50

<210>  19
<211>  40
<212>  PRT
<213>  Homo sapiens

<400>  19

Val Gly Cys Val Leu Gly Thr Cys Gln Val Gln Asn Leu Ser His Arg
1               5                   10                  15

Leu Trp Gln Leu Met Gly Pro Ala Gly Arg Gln Asp Ser Ala Pro Val
            20                  25                  30

Asp Pro Ser Ser Pro His Ser Tyr
        35                  40

<210>  20
<211>  37
<212>  PRT
<213>  Bos primigenius

<400>  20

Ala Cys Asn Thr Ala Thr Cys Val Thr His Arg Leu Ala Gly Leu Leu

1                   5                   10                  15

Ser Arg Ser Gly Gly Met Val Lys Ser Asn Phe Val Pro Thr Asn Val
            20                  25                  30

Gly Ser Lys Ala Phe
        35

<210>  21
<211>  37
<212>  PRT
<213>  Homo sapiens

<400>  21

Ala Cys Asn Thr Ala Thr Cys Val Thr His Arg Leu Ala Gly Leu Leu
1               5                   10                  15

Ser Arg Ser Gly Gly Met Val Lys Ser Asn Phe Val Pro Thr Asn Val
            20                  25                  30

Gly Ser Lys Ala Phe
        35

<210>  22
<211>  46
<212>  PRT
<213>  Homo sapiens

<400>  22

Ala Cys Asn Thr Ala Thr Cys Met Thr His Arg Leu Ala Gly Trp Leu
1               5                   10                  15

Ser Arg Ser Gly Ser Met Val Arg Ser Asn Leu Leu Pro Thr Lys Met
            20                  25                  30

Gly Phe Lys Ile Phe Asn Gly Pro Arg Arg Asn Ser Trp Phe
        35                  40                  45

<210>  23
<211>  46
<212>  PRT
<213>  Capra aegagrus hircus

<400>  23

Ala Cys Asn Thr Ala Thr Cys Met Thr His Arg Leu Ala Gly Trp Leu
1               5                   10                  15

Ser Arg Ser Gly Ser Met Val Arg Ser Asn Leu Leu Pro Thr Lys Met
            20                  25                  30

Gly Phe Lys Ile Phe Ser Gly Pro Arg Lys Asn Phe Trp Phe
        35                  40                  45

<210>  24
<211>  38
<212>  PRT
<213>  Canis lupus familiaris

<400>  24

Ser Cys Asn Ser Ala Thr Cys Val Ala His Trp Leu Gly Gly Leu Leu
1               5                   10                  15

Ser Arg Ala Gly Ser Val Ala Asn Thr Asn Leu Leu Pro Thr Ser Met
            20                  25                  30

Gly Phe Lys Val Tyr Asn
        35

<210>  25
<211>  40
<212>  PRT
<213>  Ovis aries

<400>  25

Ala Cys Asn Thr Ala Thr Cys Met Thr His Arg Leu Ala Gly Trp Leu
1               5                   10                  15

Ser Arg Ser Gly Ser Met Val Arg Ser Asn Leu Leu Pro Thr Lys Met
            20                  25                  30

Gly Phe Lys Ile Phe Ser Gly Pro
        35                  40

<210>  26
<211>  40
<212>  PRT
<213>  Bos primigenius

<400>  26

Ala Cys Asn Thr Ala Thr Cys Met Thr His Arg Leu Ala Gly Trp Leu
1               5                   10                  15

Ser Arg Ser Gly Ser Met Val Arg Ser Asn Leu Leu Pro Thr Lys Met
            20                  25                  30

Gly Phe Lys Ile Phe Asn Gly Pro
        35                  40

<210>  27

<211>  38
<212>  PRT
<213>  Sus domestica

<400>  27

Ser Cys Asn Thr Ala Thr Cys Met Thr His Arg Leu Val Gly Leu Leu
1               5                   10                  15

Ser Arg Ser Gly Ser Met Val Arg Ser Asn Leu Leu Pro Thr Lys Met
            20                  25                  30

Gly Phe Lys Val Phe Gly
        35

<210>  28
<211>  37
<212>  PRT
<213>  Equus ferus caballus

<400>  28

Ser Cys Asn Thr Ala Ser Cys Leu Thr His Arg Leu Ala Gly Leu Leu
1               5                   10                  15

Ser Ser Ala Gly Ser Met Ala Asn Ser Asn Leu Leu Pro Thr Glu Met
            20                  25                  30

Gly Phe Lys Val Ser
        35

<210>  29
<211>  47
<212>  PRT
<213>  Canis lupus familiaris

<400>  29

Ser Ser Cys Lys Asp Gly Pro Cys Val Thr Asn Arg Leu Glu Gly Trp
1               5                   10                  15

Leu Ala Arg Ala Glu Arg Met Val Lys Asn Thr Phe Met Pro Thr Asp
            20                  25                  30

Val Asp Pro Glu Ala Phe Gly His Gln His Lys Glu Leu Ala Ala
        35                  40                  45

<210>  30
<211>  39
<212>  PRT
<213>  Capra aegagrus hircus

<400>  30

Ser Cys Asn Arg Ala Thr Cys Val Thr His Lys Met Ala Gly Ser Leu
1               5                   10                  15

Ser Arg Ser Gly Ser Glu Ile Lys Arg Asn Phe Met Ser Thr Asn Val
            20                  25                  30

Gly Ser Lys Ala Phe Gly Gln
        35

<210>  31
<211>  37
<212>  PRT
<213>  Sus domestica

<400>  31

Ser Cys Asn Thr Ala Ser Cys Val Thr His Lys Met Thr Gly Trp Leu
1               5                   10                  15

Ser Arg Ser Gly Ser Val Ala Lys Asn Asn Phe Met Pro Thr Asn Val
            20                  25                  30

Asp Ser Lys Ile Leu
        35

<210>  32
<211>  47
<212>  PRT
<213>  Canis lupus familiaris

<400>  32

Ser Ser Cys Lys Asp Gly Pro Cys Val Thr Asn Arg Leu Glu Gly Trp
1               5                   10                  15

Leu Ala Arg Ala Glu Arg Met Val Lys Asn Thr Phe Met Pro Thr Asp
            20                  25                  30

Val Asp Pro Glu Ala Phe Gly His Gln His Lys Glu Leu Ala Ala
        35                  40                  45

<210>  33
<211>  37
<212>  PRT
<213>  Sus domestica

<400>  33

Ser Cys Asn Thr Ala Ile Cys Val Thr His Lys Met Ala Gly Trp Leu
1               5                   10                  15

Ser Arg Ser Gly Ser Val Val Lys Asn Asn Phe Met Pro Ile Asn Met
            20                  25                  30

Gly Ser Lys Val Leu
        35

<210>  34
<211>  47
<212>  PRT
<213>  Canis lupus familiaris

<400>  34

Ser Ser Cys Lys Asp Gly Pro Cys Val Thr Asn Arg Leu Glu Gly Trp
1               5                   10                  15

Leu Ala Arg Ala Glu Arg Met Val Lys Asn Thr Phe Met Pro Thr Asp
            20                  25                  30

Val Asp Pro Glu Ala Phe Gly His Gln His Lys Glu Leu Ala Ala
        35                  40                  45

<210>  35
<211>  47
<212>  PRT
<213>  Canis lupus familiaris

<400>  35

Ser Ser Cys Lys Asp Gly Pro Cys Val Thr Asn Arg Leu Glu Gly Trp
1               5                   10                  15

Leu Ala Arg Ala Glu Arg Met Val Lys Asn Thr Phe Met Pro Thr His
            20                  25                  30

Val Asp Pro Glu Asp Phe Gly His Gln His Lys Glu Leu Ala Ala
        35                  40                  45

<210>  36
<211>  32
<212>  PRT
<213>  Salmon

<220>
<221>  CHAIN
<222>  (1)..(6)
<223>  (CH2)5 bridge

<400>  36

Cys Ser Asn Leu Ser Thr Cys Val Leu Gly Lys Leu Ser Gln Glu Leu
1               5                   10                  15

His Lys Leu Gln Thr Tyr Pro Arg Thr Asn Thr Gly Ser Gly Thr Pro
            20                  25                  30

<210>  37
<211>  32
<212>  PRT
<213>  Artificial

<220>
<223>  General formula

<220>
<221>  MISC_FEATURE
<222>  (2)..(2)
<223>  S, G, OR A

<220>
<221>  MISC_FEATURE
<222>  (8)..(8)
<223>  V OR M

<220>
<221>  MISC_FEATURE
<222>  (11)..(11)
<223>  T OR K

<220>
<221>  MISC_FEATURE
<222>  (12)..(12)
<223>  T OR L

<220>
<221>  MISC_FEATURE
<222>  (13)..(13)
<223>  T OR S

<220>
<221>  MISC_FEATURE
<222>  (15)..(15)
<223>  D OR Q

<220>
<221>  MISC_FEATURE
<222>  (16)..(16)
<223>  F OR L

<220>
<221>  MISC_FEATURE
<222>  (17)..(17)
<223>  N OR H

<220>
<221>  MISC_FEATURE
<222>  (19)..(19)
<223>  Y, F, OR L

<220>
<221>  MISC_FEATURE
<222>  (20)..(20)
<223>  H OR Q

<220>
<221>  MISC_FEATURE

<222>  (22)..(22)
<223>  Y OR F

<220>
<221>  MISC_FEATURE
<222>  (24)..(24)
<223>  Q OR R

<220>
<221>  MISC_FEATURE
<222>  (26)..(26)
<223>  A, S, N OR D

<220>
<221>  MISC_FEATURE
<222>  (27)..(27)
<223>  I, T, OR V

<220>
<221>  MISC_FEATURE
<222>  (29)..(29)
<223>  V, S OR A

<220>
<221>  MISC_FEATURE
<222>  (31)..(31)
<223>  A, T, OR V

<220>
<221>  MISC_FEATURE
<222>  (33)..(33)
<223>  amidized homoserine, homoserine-lactone reacted with a primary
       alkyl amine containing 1 - 20 carbon atoms or an optional
       polypeptide chain and containing an amidized homoserine at the
       C-terminal.

<400>  37

Cys Xaa Asn Leu Ser Thr Cys Xaa Leu Gly Xaa Xaa Xaa Gly Xaa Xaa
1               5                   10                  15

Xaa Leu Xaa Xaa Thr Xaa Pro Xaa Thr Xaa Xaa Gly Xaa Gly Xaa Pro
            20                  25                  30

<210>  38
<211>  32
<212>  PRT
<213>  Artificial

<220>
<223>  construct

<400>  38

Cys Gly Asn Leu Ser Thr Cys Met Leu Gly Lys Leu Ser Gln Glu Leu
1               5                   10                  15

His Lys Leu Gln Thr Tyr Pro Gln Thr Ala Ile Gly Val Gly Ala Pro
            20                  25                  30

<210>  39
<211>  32
<212>  PRT
<213>  Homo sapiens

<400>  39

Cys Gly Asn Leu Ser Thr Cys Met Leu Gly Thr Tyr Thr Gln Asp Phe
1               5                   10                  15

Asn Lys Phe His Thr Phe Pro Gln Thr Ala Ile Gly Val Gly Ala Pro
            20                  25                  30

<210>  40
<211>  37
<212>  PRT
<213>  rat

<400>  40

Lys Cys Asn Thr Ala Thr Cys Ala Thr Gln Arg Leu Ala Asn Phe Leu
1               5                   10                  15

Val Arg Ser Ser Asn Asn Leu Gly Pro Val Leu Pro Pro Thr Asn Val
            20                  25                  30

Gly Ser Asn Thr Tyr
        35

<210>  41
<211>  36
<212>  PRT
<213>  Homo sapiens

<400>  41

Ala Cys Asp Thr Ala Thr Cys Val Thr His Arg Leu Ala Gly Leu Leu
1               5                   10                  15

Ser Ser Gly Gly Val Val Lys Asn Asn Phe Val Pro Thr Asn Val Gly
            20                  25                  30

Ser Lys Ala Phe
        35

<210>  42
<211>  38
<212>  PRT
<213>  artificial

<220>
<223>  sythetic construct

<400>  42

Tyr Ala Cys Asp Thr Ala Thr Cys Val Thr His Arg Leu Ala Gly Leu
1               5                   10                  15

Leu Ser Arg Ser Gly Gly Val Val Lys Asn Asn Phe Val Pro Thr Asn
            20                  25                  30

Val Gly Ser Lys Ala Phe
        35

<210>  43
<211>  37
<212>  PRT
<213>  artificial

<220>
<223>  sythetic construct

<220>
<221>  MISC_FEATURE
<222>  (2)..(2)
<223>  Cys(Et)

<220>
<221>  MISC_FEATURE
<222>  (7)..(7)
<223>  Cys(Et)

<400>  43

Ala Cys Asp Thr Ala Thr Cys Val Thr His Arg Leu Ala Gly Leu Leu
1               5                   10                  15

Ser Arg Ser Gly Gly Val Val Lys Asn Asn Phe Val Pro Thr Asn Val
            20                  25                  30

Gly Ser Lys Ala Phe
        35

<210>  44
<211>  37
<212>  PRT
<213>  artificial

<220>
<223>  synthetic construct

<220>
<221>  MISC_FEATURE
<222>  (2)..(2)
<223>  Cys(Acm)

<220>
<221>  MISC_FEATURE
<222>  (7)..(7)

<223>  Cys(Acm)

<400>  44

Ala Cys Asp Thr Ala Thr Cys Val Thr His Arg Leu Ala Gly Leu Leu
1               5                   10                  15

Ser Arg Ser Gly Gly Val Val Lys Asn Asn Phe Val Pro Thr Asn Val
            20                  25                  30

Gly Ser Lys Ala Phe
        35

<210>  45
<211>  37
<212>  PRT
<213>  Homo sapiens

<400>  45

Ala Cys Asn Thr Ala Thr Cys Val Thr His Arg Leu Ala Gly Leu Leu
1               5                   10                  15

Ser Arg Ser Gly Gly Met Val Lys Ser Asn Phe Val Pro Thr Asn Val
            20                  25                  30

Gly Ser Lys Ala Phe
        35

<210>  46
<211>  40
<212>  PRT
<213>  artificial

<220>
<223>  synthetic construct

<400>  46

Gln Leu Met Gly Pro Ala Gly Arg Gln Asp Ser Ala Pro Val Asp Pro
1               5                   10                  15

Ser Ser Pro His Ser Tyr Val Gly Cys Val Leu Gly Thr Cys Gln Val
            20                  25                  30

Gln Asn Leu Ser His Arg Leu Trp
        35                  40

<210>  47
<211>  52
<212>  PRT
<213>  Homo sapiens

<400>  47

Tyr Arg Gln Ser Met Asn Asn Phe Gln Gly Leu Arg Ser Phe Gly Cys
1               5                   10                  15

Arg Phe Gly Thr Cys Thr Val Gln Lys Leu Ala His Gln Ile Tyr Gln
            20                  25                  30

Phe Thr Asp Lys Asp Lys Asp Asn Val Ala Pro Arg Ser Lys Ile Ser
        35                  40                  45

Pro Gln Gly Tyr
    50

<210>  48
<211>  37
<212>  PRT
<213>  rat

<400>  48

Lys Cys Asn Thr Ala Thr Cys Ala Thr Gln Arg Leu Ala Asn Phe Leu
1               5                   10                  15

Val Arg Ser Ser Asn Asn Leu Gly Pro Val Leu Pro Pro Thr Asn Val
            20                  25                  30

Gly Ser Asn Thr Tyr
        35

<210>  49
<211>  37
<212>  PRT
<213>  Homo sapiens

<400>  49

<210>  50
<211>  37
<212>  PRT
<213>  cat

<400>  50

Lys Cys Asn Thr Ala Thr Cys Ala Thr Gln Arg Leu Ala Asn Phe Leu

1                   5                   10                  15

Ile Arg Ser Ser Asn Asn Leu Gly Ala Ile Leu Pro Pro Thr Asn Val
            20                  25                  30

Gly Ser Asn Thr Tyr
        35

<210>  51
<211>  37
<212>  PRT
<213>  guinea pig

<400>  51

Lys Cys Asn Thr Ala Thr Cys Ala Thr Gln Arg Leu Thr Asn Phe Leu
1               5                   10                  15

Val Arg Ser Ser His Asn Leu Gly Ala Ala Leu Leu Pro Thr Asp Val
            20                  25                  30

Gly Ser Asn Thr Tyr
        35

<210>  52
<211>  37
<212>  PRT
<213>  new world rodent

<400>  52

Lys Cys Asn Thr Ala Thr Cys Ala Thr Gln Arg Leu Thr Asn Phe Leu
1               5                   10                  15

Val Arg Ser Ser His Asn Leu Gly Ala Ala Leu Pro Pro Thr Lys Val
            20                  25                  30

Gly Ser Asn Thr Tyr
        35

<210>  53
<211>  36
<212>  PRT
<213>  Syrinan Hamster

<400>  53

Lys Cys Asn Thr Ala Thr Cys Ala Thr Gln Arg Leu Ala Asn Phe Leu
1               5                   10                  15

Val His Ser Asn Asn Leu Gly Pro Val Leu Ser Pro Thr Asn Val Gly
            20                  25                  30

Ser Asn Thr Tyr
        35

<210>  54
<211>  37
<212>  PRT
<213>  dog

<400>  54

Lys Cys Asn Thr Ala Thr Cys Ala Thr Gln Arg Leu Ala Asn Phe Leu
1               5                   10                  15

Val Arg Thr Ser Asn Asn Leu Gly Ala Ile Leu Ser Pro Thr Asn Val
            20                  25                  30

Gly Thr Asn Thr Tyr
        35

<210>  55
<211>  37
<212>  PRT
<213>  rat

<400>  55

Ser Gly Asn Thr Ala Thr Cys Val Thr His Arg Leu Ala Gly Leu Leu
1               5                   10                  15

Ser Arg Ser Gly Gly Val Val Lys Asp Asn Phe Val Pro Thr Asn Val
            20                  25                  30

Gly Ser Glu Ala Phe
        35

<210>  56
<211>  37
<212>  PRT
<213>  rat

<400>  56

Ser Gly Asn Thr Ala Thr Cys Val Thr His Arg Leu Ala Asn Leu Leu
1               5                   10                  15

Ser Arg Ser Gly Gly Val Val Lys Asp Asn Phe Val Pro Thr Asn Val
            20                  25                  30

Gly Ser Lys Ala Phe
        35

## Description

The present invention relates to materials and methods for the treatment of diabetes (Type I and Type II) and metabolic syndrome.

Worldwide, there are about 250 million diabetics and the number is projected to double in next two decades. Over 90% of this population suffers from type 2 diabetes mellitus (T2DM). It is estimated that only 50-60% of persons affected with T2DM or in stages preceding overt T2DM are currently diagnosed.

T2DM is a heterogeneous disease characterized by abnormalities in carbohydrate and fat metabolism. The causes of T2DM are multi-factorial and include both genetic and environmental elements that affect β-cell function and insulin sensitivity in tissues such as muscle, liver, pancreas and adipose tissue. As a consequence impaired insulin secretion is observed and paralleled by a progressive decline in β -cell function and chronic insulin resistance. The inability of the endocrine pancreas to compensate for peripheral insulin resistance leads to hyperglycaemia and onset of clinical diabetes. Tissue resistance to insulin-mediated glucose uptake is now recognized as a major pathophysiologic determinant of T2DM.

A success criterion for an optimal T2DM intervention is the lowering of blood glucose levels, which can be both chronic lowering of blood glucose levels and increased ability to tolerate high glucose levels after food intake, described by lower peak glucose levels and faster clearance. Both of these situations exert less strain on β-cell insulin output and function.

An approach towards T2DM control is the use of incretin hormones, Glucagon-like Peptide 1 (GLP-1) and glucose-dependent insulinotropic polypeptide (GIP), which are produced by the endocrine cells of the intestine following ingestion of food, and stimulate insulin production. GLP-1 is ineffective as a clinical treatment for diabetes as it has a very short half-life in vivo. Pharmacological synthetic examples of incretins are Exenatide and Liraglutide that displays biological properties similar to human GLP-1, but offer longer half-life. However these GLP-1 analogues are associated with several adverse effects, such as rare-but-dangerous side effect of pancreatitis, and cardiovascular effects.

EP0309100, relating to a filing in 1988 indicates that amylin may be secreted along with insulin and on the strength of that suggests a parenteral composition for treating type I diabetes comprising an amylin agonist such as amylin itself and insulin. The role suggested for amylin is to prevent hypoglycaemia occurring as an effect of insulin treatment. Amylin is said to reduce the rate of glycogen synthesis.

Gomez-Foix et al reported that amylin and CGRP exerted anti-insulin effects in isolated rat hepatocytes, supplementing previous reports of amylin and CGRP inhibiting insulin secretion.

WO89/06135 suggests that compounds blocking the effect of amylin (amylin antagonists) are useful in treating type II diabetes. Compounds that may be used as amylin antagonists are cross-linked versions of amylin agonists. Amylin is said to have the effects of casing β cells in the pancreas to release less insulin and to cause also a major reduction in both basal and insulin-stimulated glycogen synthesis in skeletal muscle by causing muscle cells to ignore the insulin signal.

WO93/10146 discloses certain amylin agonistsas parenteral agents for treating type I diabetes and for treating hypoglycaemia. It is said that in type I diabetes, amylin levels are severely reduced or non-existent. Again, the suggestion is that amylin agonists will serve to prevent low blood glucose induced by insulin treatment.

EP0717635B1/ WO95/07098 acknowledges that amylin has hyperglycaemic effects but discloses that it also can reduce gastric motility and slow gastric emptying, so reducing rather than increasing post-prandial plasma glucose levels. Accordingly, this document teaches amylin agonists for this purpose, but excludes calcitonins specifically.

US7399744 discloses the use of amylin or amylin agonists for modulating body fat. In tests, amylin delivered by osmotic pump to rats caused a reduction in weight gain in rats fed a high fat diet. Calcitonins, including teleost calcitonins are given as an example of a suitable agonist, but no data relating to their use is given.

Thus, injectable amylin is now seen as a viable approach for T2DM glycemic control as it potently inhibits postprandial glucagon secretion and gastric emptying, decreases food intake, and thereby physiologically regulates carbohydrate absorption. One amylin analogue, Pramlintide (or Symlin), is approved for treatment of type 1 and type 2 diabetics, who also use insulin. Pramlintide treatment lowers average blood sugar levels, and substantially reduces the pathological rise in postprandial blood sugar in diabetics. Pramlintide treatment also results in weight loss, and allows patients to use less insulin. US 2009/0018053 briefly proposes enterically coated formulations of pramlintide for release thereof in the gastrointestinal tract for diabetes treatment.

Amylin is a part of the calcitonin family consisting of calcitonin, α-calcitonin gene-related peptide (aCGRP), βCGRP, adrenomodullin, and amylin. This unique group of peptides shares a conserved tertiary structure with an N-terminal disulfide-bridged ring [Hay 2003 Br J Pharmacol] In mammals, these peptides signal through two closely related type II GPCRs (Calcitonin Receptor and Calcitonin Receptor-like Receptor) and three unique receptor activity-modifying proteins (RAMPs) [Hay Regul Pept 2003]. Thereby the use of any of these small signalling molecules may elicit more pleiotropic effects on multiple organs, due to receptor sharing and divergent evolution.

Calcitonin (CT) is a natural peptide hormone produced by parafollicular cells (C-cells) in the thyroid gland and secreted in response to excess calcium in the serum. CT reduces osteoclastic resorption by direct binding to its receptors on the osteoclast cell surface. CT is approved for the treatment of osteoporosis, malignancy-associated hypercalcemia, Paget's disease, which all involve accelerated bone turnover. An oral form of calcitonin has recently been described in the literature.

There have been numerous reports of a diabetogenic effect produced by administration of calcitonin in animals and in man, except perhaps in Type I diabetes sufferers.

Looking first at animal and *in vitro* studies, Lupulescu reported in 1974 that large doses (600 MRC U per month or 50 MRC U single dose) of synthetic salmon calcitonin administered by injection to rabbits produced a marked decrease in glucose level measured after fasting, whether the administration was for just once or three times a day for one month. The mechanism for this was not understood and no study was made of the effect on glucose levels after a glucose tolerance test or during/following normal feeding. They acknowledge that no similar results were seen by Aldred et al 1968 when administering porcine calcitonin to rats, rabbits, or mice.

Greeley in 1989 reported that intracerebroventricular administration of salmon calcitonin in rats increased glucose stimulated release of insulin, however glucose plasma levels do not depend exclusively on insulin and other studies (below) suggest that in addition to increasing insulin, calcitonin also increases glucagon levels which prevail over the insulin increase to produce an increased glucose level.

Pittner in 1997 reported that little effect when amylin, calcitonin gene related peptide or rat or salmon calcitonin were tested on rat hepatocytes and other cells.

Young et al in 1995 reported that in rats and mice, salmon calcitonin as an intravenous bolus injection produced elevation of fasting plasma glucose, rat calcitonin having a lesser effect. In a hypoglycaemic rat, calcitonin produced a greater glycemic recovery than glucagon and in combination synergistically increased the glycemic recovery produced by glucagon.

Young in 2005 summarised at length the effects of amylin and to a lesser extent salmon calcitonin on glucose and lactate levels in animals. He reported that both amylin and salmon calcitonin parenterally administered to fasting animals produced a rise in glucose levels, calcitonin producing the stronger glucose raising effect, but that this effect was mild or absent in humans. He reported also that amylin parenterally administered shortly before an oral glucose tolerance test in rats reduced the rise in glucose. In this respect therefore it is clear that amylin and salmon calcitonin behave differently in the rat, as it is well established that parenteral calcitonin produces an increased glucose maximum in an oral glucose tolerance test.

Chelikani et al in 2007 reported that chronic administration of anorexigenic substances to animals by infusion or repeated injection produces no lasting effect on food intake or body weight. Although acute parenteral administration of salmon calcitonin potently induced reduction in short term food intake in rats or mice, the effect lasted only for 3 days.

Bello et al in 2008 reported that parenteral salmon calcitonin did however reduce food intake in rhesus monkeys throughout a 5 day administration period.

Looking next at work in humans, Ziegler et al (1972)reported that in healthy test persons an infusion of synthetic human calcitonin produced a significant impairment of glucose assimilation and insulin output.

Blahos et al in 1976 reported that intramuscular salmon calcitonin in healthy volunteers inhibited decrease of blood glucose during a morning fast and impaired a glucose tolerance test.

Petralito et al (1979) reported that in cases of latent diabetes in humans, an infusion of salmon calcitonin proved capable of reducing the serum level of insulin and increasing the blood sugar.

Giugliano et al 1980 reported that whilst it was previously established that acute calcitonin administration impairs glucose tolerance in normal, obese and pre-diabetic human subjects, salmon calcitonin administered by intravenous infusion to insulin dependent diabetic patients abolished the glucose rise normally seen in an arginine tolerance test, with an immediate rebound in glucose level once the infusion of calcitonin stopped.

Gattereau et al (1980) reported that injection of either salmon calcitonin or human calcitonin administered to Paget's disease of the bone patients provoked a moderate immediate rise in serum glucose and a slight decrease in serum insulin.

Passariello et al (1981) reported that intra-muscular administration of synthetic salmon calcitonin to humans produced a deterioration in response to a glucose tolerance test in normal humans and causes a further impairment of glucose tolerance in subjects with IGT. There was observed an approximately doubling of the integrated glucose areas under plasma glucose curves.

Starke et al in 1981 reported that in normal human volunteers, infused salmon calcitonin produced a fall in circulating glucagon levels and a fall in serum insulin, with the net effect the expected fall in glucose due to decreased glucagon being masked by the effect of the fall in insulin. However, in insulin dependent diabetics, salmon calcitonin produced a fall in glucose level along with the fall in glucagon.

Giugliano et al (1982) reported the results of long term (2 months) administration of 100 MRC units per day of salmon calcitonin to patients with Paget's disease of the bone or significant osteoporosis. Glucose tolerance did not deteriorate significantly with treatment (although a non-significant rise in peak glucose is seen), but there was a significant increase in basal plasma glucose. The first ten minutes of insulin response to glucose administration was attenuated by calcitonin.

Giustina et al (1985) investigated the effect of short term (15 days) intra muscular administration of 100 MRC units twice daily of salmon calcitonin in patient's with Paget's disease of the bone, idiopathic osteoporosis or Sudeck's osteodystrophy. Some of the patient's were non insulin-dependent diabetics also receiving anti-diabetes therapy. The short term i.m. sCT treatment was not seen to cause appreciable change in carbohydrate metabolism after a mixed meal stimulus. There was a non-significant reduction of glucose level observed during the night in the three diabetic patients included.

Zofkova (1987 - Exp. Clin. Endocrinol.) found that 100 U of calcitonin given by i.v. infusion in the course of an OGTT caused a persistence of hyperglycaemia, after an initially slower rise. This was explained as being due to interference with glucose absorption and metabolism in the liver. An inhibitory action of calcitonin on insulin secretion was also observed.

Zofkova (1987 - Horm. Metabol. Res.) looked at the effect of two doses (50 and 100 U) of salmon calcitonin on glucose blood levels in healthy volunteers and found similar results to those just described above at both dose levels.

Mangiafico (1988) found that a combination of nifedipine with salmon calcitonin administered at 100 U/day produced a statistically significant rise in blood sugar in subjects with hypertension or with non insulin-dependent diabetes or impaired glucose tolerance at all times in a 3 week program.

Jonderko (1989) found that post-prandial insulin release was abolished by 62.26 pmol/kg of salmon calcitonin in patients with a duodenal ulcer with a parallel rise in serum glucose during calcitonin infusion.

Young (2005) reported that in the context of a meal, administration of amylin suppressed a rise in plasma glucose in rats, dogs and humans, but that in the absence of a meal, administration of amylin was associated with a rise in plasma glucose in rodents, but not in humans.

In summary, the studies of the effect of parenteral administration to humans suffering from non-insulin dependent diabetes or impaired glucose tolerance referred to above demonstrate conclusively that such calcitonin administration is not of therapeutic benefit.

We investigated whether enterally administered CT was able to improve a hyperglycaemic state and provide improved glycemic control. We compared the putative effect of an oral formulation of salmon CT to that of the well-established PPAR-γ agonist rosiglitazone in diet induced obese rats (DIO), which exhibit several characteristics of T2DM. Finally, we investigated the effect of calcitonin on glucose handling in healthy adult rats. We have unexpectedly found that oral administration of a calcitonin produces essentially the opposite effect to that previously disclosed for injected or infused calcitonin, as described and illustrated below. In contrast, insulin for example which is also available for oral administration or injection provides the same kind of effect by whichever route is used.

Accordingly, the present invention provides a pharmaceutical formulation for enteral administration for treating type I diabetes, type II diabetes, or metabolic syndrome, or for mitigating insulin resistance, or for reducing an undesirably high fasting serum glucose level, or for reducing an undesirably high peak serum glucose level, or for reducing an undesirably high peak serum insulin level, which formulation comprises an active compound which is a calcitonin family member other than amylin, a modified calcitonin family member other than a modified amylin, or a calcitonin receptor agonist. The formulation may comprise also a carrier serving to enable effective enteral administration of said active compound.

Preferably, said formulation is formulated for oral administration to the digestive tract.

Preferably, the active compound is a calcitonin, most preferably salmon calcitonin.

The active compound may be a modified calcitonin family member having at least 75% amino acid homology with a calcitonin member other than amylin and being modified with respect to said calcitonin family member by addition, substitution or deletion of amino acids and retaining the ability to bind and to activate the calcitonin receptor.

Preferably, said carrier comprises 5-CNAC.

The invention includes a method of treatment of type I diabetes, Type II diabetes or metabolic syndrome comprising enteral administration to a patient in need thereof for treatment of a said condition of a pharmaceutically effective amount of pharmaceutical formulation comprising an active compound which is a calcitonin family member other than amylin, a modified calcitonin family member other than a modified amylin, or a calcitonin receptor agonist, and optionally a carrier serving to enable effective enteral administration of said active compound. Said method may include a preliminary step of determining whether the patient suffers from a said condition, and/or a subsequent step of determining to what extent said treatment is effective in mitigating the condition in said patient, e.g. in each case, carrying out an oral glucose tolerance test or a resting blood sugar level.

For improved control over the weight of the patient, to produce a loss of weight or an avoidance of weight gain, the active compound is preferably administered at least twice per day, e.g. from 2-4 times per day. Formulations of the active compound may contain a unit dosage appropriate for such an administration schedule. The active compounds may be administered with a view to controlling the weight of a patient undergoing treatment for diabetes or metabolic syndrome.

Oral enteral formulations are for ingestion by swallowing for subsequent release in the intestine below the stomach, and hence delivery via the portal vein to the liver, as opposed to formulations to be held in the mouth to allow transfer to the bloodstream via the sublingual or buccal routes.

Without being bound by the theory, we speculate that the striking change in the effect of calcitonin according to whether it is parenterally administered or enterally administered that we report herein may be due to the enteral administration bringing the calcitonin immediately to the liver via the portal vein following gastric absorption, leading to receptors in the liver being exposed to a much higher concentration of calcitonin than when calcitonin is administered iv or im. The direct route followed by enterally administered calcitonin to the liver enables an anti-hyperglycaemic effect to be obtained despite the short half life of calcitonin (similar to that of GLP-1). The mechanism underlying the reversal in effect between injected and oral administration of calcitonin may be that in a sufficiently high concentration calcitonin is capable of acting as an agonist at receptors normally acted on by amylin, and thus can produce an amylin like effect, whereas at lower concentrations calcitonin is effective only at other receptors which produce a hyperglycaemic effect. Other explanations are however possible. For instance, it may be that administered orally, these agents act directly on calcitonin receptors different from those on which acts injected calcitonin, for instance on receptors in the intestinal tract itself.

Calcitonins are highly conserved over a wide range of species. The sequences of examples of calcitonins are set out below:
Human CGNLSTCMLGTYTQDFNKFHTFPQTAIGVGAP SEQ ID NO:1
Salmon CSNLSTCVLGKLSQELHKLQTYPRTNTGSGTP SEQ ID NO:2
Mouse CGNLSTCMLGTYTQDLNKFHTFPQTSIGVEAP SEQ ID NO:3
Chicken CASLSTCVLGKLSQELHKLQTYPRTDVGAGTP SEQ ID NO:4
Eel CSNLSTCVLGKLSQELHKLQTYPRTDVGAGTP SEQ ID NO:5
Rat CGNLSTCMLGTYTQDLNKFHTFPQTSIGVGAP SEQ ID NO:6
Horse CSNLSTCVLGTYTQDLNKFHTFPQTAIGVGAP SEQ ID NO:7
Canine-1 CSNLSTCVLGTYSKDLNNFHTFSGIGFGAETP SEQ ID NO:8
Canine-2 CSNLSTCVLGTYTQDLNKFHTFPQTAIGVGAP SEQ ID NO:9
Porcine CSNLSTCVLSAYWRNLNNFHRFSGMGFGPETP SEQ ID NO:10

Accordingly, a preferred material for use in the present invention has the general formula:
CX¹X²LSTCX³LX⁴X⁵X⁶X⁷X⁸X⁹X¹⁰X¹¹X¹²X¹³X¹⁴X¹⁵X¹⁶X¹⁷X¹⁸X¹⁹X²⁰X²¹GX²²X²³X²⁴P
SEQ ID NO:11
wherein:
X¹ is A, G, or S; preferably S;
X² is N or S; preferably N;
X³ is M or V; preferably V;
X⁴ is G or S; preferably G;
X⁵ is T, K, or A; preferably T or K; most preferably K;
X⁶ is L or Y; preferably L;
X⁷ is T, S, or W; preferably T or S; most preferably S;
X⁸ is Q, K, or R; preferably Q;
X⁹ is D, E, or N; preferably D or E; most preferably E;
X¹⁰ is F or L; preferably L;
X¹¹ is N or H; preferably H;
X¹² is K or N; preferably K;
X¹³ is F or L; preferably L;
X¹⁴ is H or Q; preferably Q;
X¹⁵ is T or R; preferably T;
X¹⁶ is F or Y; preferably Y;
X¹⁷ is P or S; preferably P;
X¹⁸ is Q, G or R; preferably Q or R; most preferably R;
X¹⁹ is T, I or M; preferably T;
X²⁰ is A, N, D, S, or G; preferably N;
X²¹ is I, T, V or F; preferably T,
X²² is V, S, A, or P; preferably V, S, or A; most preferably S;
X²³ is G or E; preferably G;
X²⁴ is A or T; preferably T.

Accordingly, it is preferred that the material is of the formula:
CSNLSTCVLGX⁵LX⁷QX⁹LHKLQTYPX¹⁸TNTGX²²GTP
SEQ ID NO:12
wherein
X⁵ is T or K; more preferably K;
X⁷ is T or S; more preferably S;
X⁹ is D or E; more preferably E;
X¹⁸ is Q or R; more preferably R;
X²² is V, S, or A; more preferably S.

Salmon calcitonin is most preferred.

Furthermore, calcitonin is recognised to be a member of a family of peptide hormones comprising amylin, calcitonin gene related peptide, adrenomedullin, intermedin, calcitonin gene related peptide II and calcitonin receptor stimulating peptide-1, -2, -3, -4 and -5. Of these CRSP-1 is preferred for use in this invention over the other calcitonin receptor stimulating peptides.

These calcitonin family members share a significant degree of amino acid sequence homology as well as structural similarities. These include a disulphide bridged loop of 6 or 7 amino acids at the amino terminus, a C-terminally amidated aromatic residue present at the carboxy terminus, and a region of predicted amphipathic α-helical structure from residues 8-18 or 8-22.

Amino acid sequences for the other members of calcitonin family from various species are:

### AMYLIN

Human KCNTATCATQRLANFLVHSSNNFGAILSSTNVGSNTY SEQ ID NO:13
Mouse KCNTATCATQRLANFLVRSSNNLGPVLPPTNVGSNTY SEQ ID NO:14

### CALCITONIN GENE RELATED PEPETIDE

Human ACDTATCVTHRLAGLLSRSGGVVKNNFVPTNVGSKAF SEQ ID NO:15
Porcine SCNTATCVTHRLAGLLSRSGGMVKSNFVPTDVGSEAF SEQ ID NO:16

### ADRENOMEDULLIN

Human TQAQLLRVGCVLGTCQVQNLSHRLWQLMGPAGRQDSAPVDPSSPHSY
   SEQ ID NO:17
   - Porcine: YRQSMNNFQGLRSFGCRFGTCTVQKLAHQIYQFTDKDKDGVAPRSKISPQGY
   SEQ ID NO:18

### INTERMEDIN

Human VGCVLGTCQVQNLSHRLWQLMGPAGRQDSAPVDPSSPHSY
   SEQ ID NO:19

### CALCITONIN GENE-RELATED PEPTIDE II

Bovine ACNTATCVTHRLAGLLSRSGGMVKSNFVPTNVGSKAF SEQ ID NO:20
Human ACNTATCVTHRLAGLLSRSGGMVKSNFVPTNVGSKAF SEQ ID NO:21

### CALCITONIN RECEPTOR STIMULATING PEPTIDE-1

Human ACNTATCMTHRLAGWLSRSGSMVRSNLLPTKMGFKIFNGPRRNSWF
   SEQ ID NO:22
Goat ACNTATCMTHRLAGWLSRSGSMVRSNLLPTKMGFKIFSGPRKNFWF
   SEQ ID NO:23
Canine SCNSATCVAHWLGGLLSRAGSVANTNLLPTSMGFKVYN
   SEQ ID NO:24
Ovine ACNTATCMTHRLAGWLSRSGSMVRSNLLPTKMGFKIFSGP
   SEQ ID NO:25
Bovine ACNTATCMTHRLAGWLSRSGSMVRSNLLPTKMGFKIFNGP
   SEQ ID NO:26
Porcine SCNTATCMTHRLVGLLSRSGSMVRSNLLPTKMGFKVFG
   SEQ ID NO:27
Equine SCNTASCLTHRLAGLLSSAGSMANSNLLPTEMGFKVS
   SEQ ID NO:28

### CALCITONIN RECEPTOR STIMULATING PEPTIDE-2

Canine SSCKDGPCVTNRLEGWLARAERMVKNTFMPTDVDPEAFGHQHKELAA
   SEQ ID NO:29
Caprine SCNRATCVTHKMAGSLSRSGSEIKRNFMSTNVGSKAFGQ
   SEQ ID NO:30
Porcine SCNTASCVTHKMTGWLSRSGSVAKNNFMPTNVDSKIL SEQ ID NO:31

### CALCITONIN RECEPTOR STIMULATING PEPTIDE-3

Canine SSCKDGPCVTNRLEGWLARAERMVKNTFMPTDVDPEAFGHQHKELAA
   SEQ ID NO:32
Porcine SCNTAICVTHKMAGWLSRSGSVVKNNFMPINMGSKVL
   SEQ ID NO:33

### CALCITONIN RECEPTOR STIMULATING PEPTIDE-4

Canine SSCKDGPCVTNRLEGWLARAERMVKNTFMPTDVDPEAFGHQHKELAA
   SEQ ID NO:34

### CALCITONIN RECEPTOR STIMULATING PEPTIDE-5

Canine SSCKDGPCVTNRLEGWLARAERMVKNTFMPTHVDPEDFGHQHKELAA
   SEQ ID NO:35

It may be noted that although expressed by different genes and being derived from differing precursor peptides, the mature peptides CRSP-2, -3 and -4 from the dog are the same.

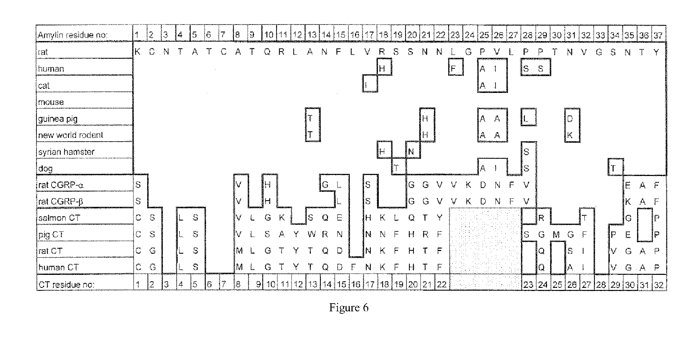

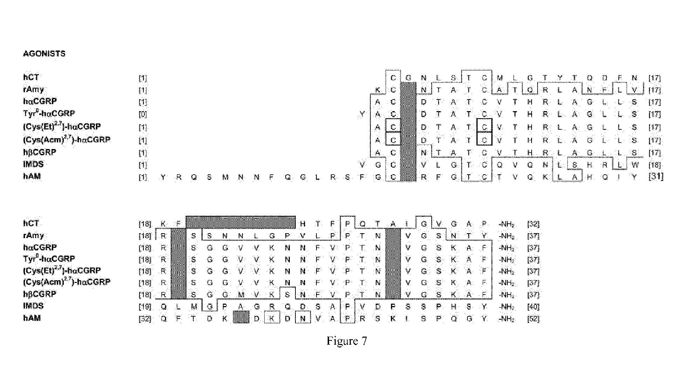

The sequences of several members of the calcitonin family are also set out in Figures 6 and 7. In Figure 6, a blank indicates that the relevant amino acid is the same as shown in the sequence for rat amylin, a printed amino acid code indicates that the sequence has that amino acid at the indicated location, and the greyed out box indicates that the calcitonins have no amino acids corresponding to amylin residues 23-27. In Figure 7, boxed areas are completely homologous (except for deletions) and greyed areas indicate that no amino acids are present at those location in the given sequences.

The different family members are to a significant degree capable of binding and activating the same receptors activated by calcitonin, i.e. capable of acting as agonists of the calcitonin receptor (CT receptor). Unlike most G-protein coupled receptors, the CT receptor can be modified by binding to one of three single-transmembrane-spanning receptor activity modifying proteins (RAMPs).

In this specification, the term 'CT receptor agonist' refers to any compound, but especially a peptide, capable of binding and activating the CT receptor in a manner demonstrable by at least one of the 'test protocols' as defined below.

The term 'calcitonin family member' refers to any one of calcitonin, amylin, calcitonin gene related peptide, adrenomedullin, intermedin, calcitonin gene related peptide II and calcitonin receptor stimulating peptide-1 as naturally occurring in any species.

The term 'modified calcitonin family member' refers to a compound having an amino acid sequence of any calcitonin family member modified with respect to the native sequence, but such that the compound in question is a CT receptor agonist. Modifications may be made for various reasons, including so as to increase the agonist effect of the compound on the CT receptor, to increase the biological half life of the compound, or to assist in the formulation of the compound for pharmaceutical use such as by increasing its storage stability.

The names of the individual calcitonin family members, e.g. 'calcitonin', refer to any naturally occurring such family member from any species, including each of the species for which calcitonin sequences are set out above, unless otherwise indicated.

The term 'modified' followed by the name of an individual calcitonin family member refers to a compound having the amino acid sequence of the calcitonin family member in question modified with respect to the native sequence, but such that the compound in question is a CT receptor agonist.

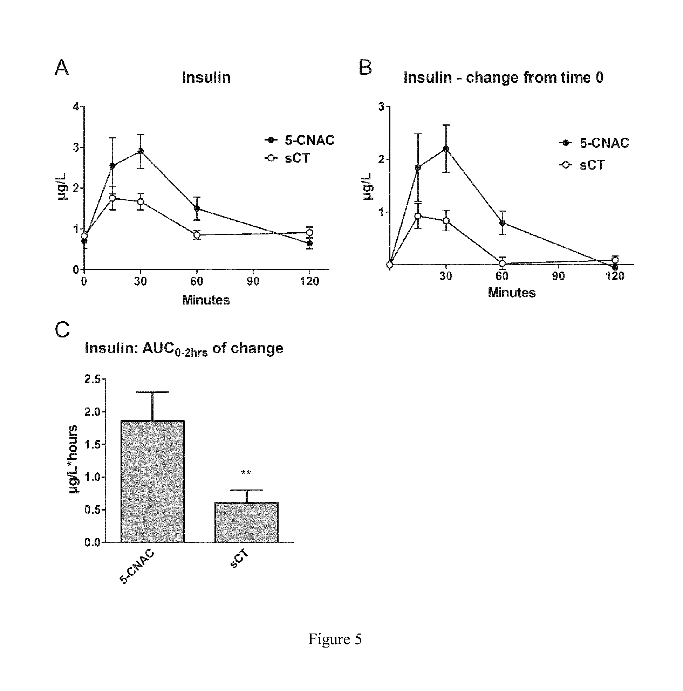

Modified calcitonin family members for use in the invention exclude modified amylin and should therefore have not more than 50% homology with amylin, preferably not more than 30%. It will be observed from Figure 5, that salmon calcitonin has only 10 of the 37 amino acids of calcitonin in its 32 amino acid make up and therefor has 10/32 * 100 % homology with amylin, i.e. 27%. Modifications of calcitonin to the extent that it becomes 'too amylin like' are excluded.

Subject to the above amino acid sequence modifications in modified calcitonin family members may be by addition, deletion, or substitution with natural or non-natural amino acids. Preferably, the modified sequence is at least 75% homologous, more preferably at least 90%, more preferably at least 95% homologous with a native sequence of the calcitonin family member in question.

Accordingly, the invention includes a formulation wherein the active compound is a modified calcitonin member having at least 75% amino acid homology with a calcitonin member other than amylin and being modified with respect to said calcitonin family member by addition, substitution or deletion of amino acids and retaining the ability to bind and to activate the calcitonin receptor.

Amongst species, the order of preference for calcitonin family members for use in the invention is teleost > avian > non-human mammalian > human.

The calcitonin family member, if naturally occurring may be natural or may be synthesised (including recombinant), and if not naturally occurring may be synthesised.

Salmon calcitonin is especially preferred amongst the naturally occurring calcitonins.

### Test Protocols

To determine whether a candidate compound is a calcitonin receptor agonist, four test protocols have been developed.

COS-7 cells are gown to 80% confluence in 75 cm² flasks. pcDNA3.1 (+) from Invitrogen is used for transfecting the cells with receptor encoding sequences. Cells are transfected with 300 ng of pcDNA-CTR construct alone (Test Protocol 1), or are cotransfected with 300 ng of pcDNA-CTR construct and 1 µg of pcDNA-RAMP-1 (Test Protocol 2), or pcDNA-RAMP-2 (Test Protocol 3), or pcDNA-RAMP-3 (Test Protocol 4), using 7.8 µL of FuGene 6 reagent.

The sequence of CTR DNA incorporated into the pcDNA-CTR construct is that given for the human calcitonin receptor cDNA in gene bank number CALCR: NM 001742. The sequences of RAMP 1 DNA, RAMP 2 DNA and RAMP 3 DNA incorporated into the pcDNA-RAMP constructs are the human RAMP sequences given in the following specific gene bank numbers.
RAMP1: NM 005855
RAMP2: NM 005854
RAMP3: NM 005856

After 48 hours cells are lifted by trypsin treatment, and centrifuged at 500 x g, and resuspended in cyclase buffer (DMEM containing 0.1% (wt/vol) BSA and 1 mM IBMX). Cells (5 x 105) are aliquoted into 1.5 ml Eppendorf tubes and preincubated for 20 min at 37 °C. Cells are subsequently incubated for 18 min in the absence (basal) or presence of increasing concentrations of agonists: 100 micromolar, 1 micomolar 0.01 micromolar. Forskolin 1mM is included to determine maximal cAMP accumulation for this system as positive control.

Following incubation, the reactions quantification of the intracellular second messenger cAMP is performed according to the cAMP-EIA kit protocol (Amersham Biosciences, US).

A test compound is considered to be a CTR agonist if it induces production of cAMP in any one of the four test protocols, at a concentration of 10 micromolar, by 50% more than the vehicle control (DMEM containing 0.1% (wt/vol) BSA and 1 mM isobutylmethylxanthine (IBMX)). sCT will produce (at least in test protocol 1) more than 10 fold induction compared to the negative control when used at even 1 micromolar. Preferably therefore, a compound for use in the invention provides at least a 100% (i.e. 2x) induction in at least one of the test protocols, preferably at least a 5 fold induction. Alternatively, a compound for use in the invention provides at least 25% of the induction given by sCT in at least one said protocol, preferably at least 50% of the induction given by sCT.

Preferred CTR agonists produce a positive result in at least two of said assays (preferably test protocols 1 and 2 or 1 and 4), preferably three test protocols (preferably 1, 2 and 4) and most preferably all four test protocols. Alternatively, it is preferred that the response provided by the test compound in Test Protocol 1 is at least 25%, more preferably at least 50%, still more preferably at least 100%, greater than the response provided by the test compound in any of Test Protocols 2-4.

Examples of modified calcitonins which may be used in this invention are to be found in US 5,536,812. Thus, the C-terminal proline-amide of the calcitonin may be substituted (replaced) with homoserine amide (Hse-NH₂). Salmon or eel calcitonins thus modified are particularly preferred.

Calcitonins or other calcitonin family members may be modified as taught in GB 1,590,645 by replacing the Cys-Cys ring element with a more stable structure such as is provided by replacing the first and the second of these Cys residues (normally the first and seventh amino acids) with aminosuberic acid, so that sCT becomes:

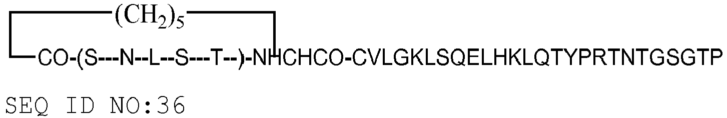

As described in EP 0464549, calcitonin analogue peptides acting as calcitonin receptor agonists may be of the general formula:
CX¹NLSTCX²LGX³X⁴X⁵GX⁶X⁷X⁸LX⁹X¹⁰TX¹¹PX¹²TX¹³X¹⁴GX¹⁵GX¹⁶P-X¹⁷ SEQ ID NO:37
wherein X¹ is Ser, Gly or Ala; X² is Val or Met; X³ is Thr or Lys; X⁴ is Tyr or Leu; X⁵ is Thr or Ser; X⁶ is Asp or Glu; X⁷ is Phe or Leu; X⁸ is Asn or His; X⁹ is Tyr, Phe or Leu; X¹⁰ is His or Gln; X¹¹ is Tyr or Phe; X¹² is Gln or Arg; X¹³ is Ala, Ser, Asn or Asp; X¹⁴ is Ile, Thr or Val; X¹⁵ is Val, Ser or Ala; X¹⁶ is Ala, Thr or Val; and X¹⁷ is amidized homoserine, homoserine-lactone reacted with a primary alkyl amine containing 1 - 20 carbon atoms or an optional polypeptide chain and containing an amidized homoserine at the C-terminal.

Alternatively, a calcitonin analogue may have the sequence PO-1 (CGNLSTCMLGKLSQELHKLQTYPQTAIGVGAP-NH2 SEQ ID NO:38), having both the N- and C-terminal ten amino acid sequences as those of human calcitonin, and the 12 amino acid central region that of salmon calcitonin, or PO-23 ([cyclo-Aspl, Lys7]-[des-Gly2]-[Leu8]-PO-1), or PO-29 ([Asp15, Asn17 , Phe19, His20]-PO-23). PO-23 was has the N-terminal Cys-Cys S-S bond of PO-1 replaced with a ring structure composed of an Asp-Lys peptide bond to enhance physicochemical stability. PO-29 the central area of the PO-23 molecule modified to more closely mimic human calcitonin.

Many other modified calcitonins having calcitonin receptor agonist properties are known in the literature.

Calcitonin receptor agonists included for use in this invention include 'small molecule' (non-peptide) agonists. These are preferably such as to satisfy the classical rules for druggability, and so preferably have at least 4 out of 5 of MW ≤ 500, logP (logarithm of its partition coefficient between n-octanol and water log (c_{octanol}/C_{water})) ≤ to 5, H-bond donors ≤ 5, H-bond acceptors (sum of N and O atoms) ≤ 10, and optionally one or both of polar surface area ≤ 140 A² (or Sum of H-bond donors and acceptors ≤ 12) and rotatable bonds ≤ 10.

Suitable dosage forms for use in the invention include tablets, minitablets, capsules, granules, pellets, powders, effervescent solids and chewable solid formulations. Such formulations may include gelatin which is preferably hydrolysed gelatin or low molecular weight gelatin. Such formulations may be obtainable by freeze drying a homogeneous aqueous solution comprising calcitonin or a fragment or conjugate thereof and hydrolysed gelatin or low molecular weight gelatin and further processing the resulting solid material into said oral pharmaceutical formulation, and wherein the gelatin may have a mean molecular weight from 1000 to 15000 Daltons. Such formulations may include a protective carrier compound such as 5-CNAC or others as disclosed herein.

Whilst oral formulations such as tablets and capsules are preferred, compositions for use in the invention may take the form of suppositories or the like. The oral delivery of calcitonins, e.g. salmon calcitonin, is generally the delivery route of choice since it is convenient, relatively easy and generally painless, resulting in greater patient compliance relative to other modes of delivery. However, biological, chemical and physical barriers such as varying pH in the gastrointestinal tract, powerful digestive enzymes, and active agent impermeable gastrointestinal membranes, makes oral delivery of calcitonins, e.g. salmon calcitonin, to mammals problematic, e.g. the oral delivery of calcitonins, which are long-chain polypeptide hormones secreted by the parafollicular cells of the thyroid gland in mammals and by the ultimobranchiai gland of birds and fish, originally proved difficult due, at least in part, to the insufficient stability of calcitonin in the gastrointestinal tract as well as the inability of calcitonin to be readily transported through the intestinal walls into the blood stream. Suitable oral formulations are however described below.

Calcitonin and other family members may be formulated for enteral, especially oral, administration by admixture with a suitable carrier compound. Administered orally by itself or in aqueous solution/suspension, it is ineffective for producing bone saving effects. Suitable carrier compounds include those described in US 5,773,647 and US5866536 and amongst these, 5-CNAC (N-(5-chlorosalicyloyl)-8-aminocaprylic acid, commonly as its disodium salt) is particularly effective. Other preferred carriers or delivery agents are SNAD (sodium salt of 10-(2-Hydroxybenzamido)decanoic acid) and SNAC (sodium salt of N-(8-[2-hydroxybenzoyl]amino)caprylic acid).

In addition, WO 00/059863 discloses the disodium salts of formula I 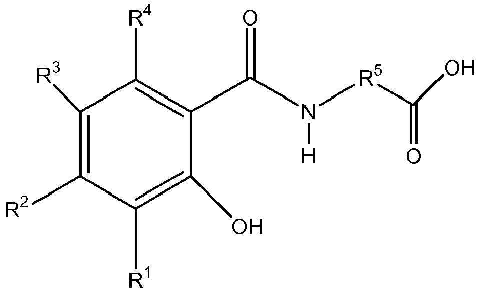 wherein
R¹, R², R³, and R⁴ are independently hydrogen, -OH, -NR⁶R⁷, halogen, C₁-C₄ alkyl, or C₁-C₄ alkoxy;
R⁵ is a substituted or unsubstituted C₂-C₁₆ alkylene, substituted or unsubstituted C₂-C₁₆ alkenylene, substituted or unsubstituted C₁-C₁₂ alkyl(arylene), or substituted or unsubstituted aryl (C₁-C₁₂ alkylene); and R⁶ and R⁷ are independently hydrogen, oxygen, or C₁-C₄ alkyl; and hydrates and solvates thereof as particularly efficacious for the oral delivery of active agents, such as calcitonins, e.g. salmon calcitonin, and these may be used in the present invention.

Preferred enteric formulations of salmon calcitonin and optionally micronised 5-CNAC may be as described in WO2005/014031.

Calcitonin and other family members may be formulated for oral administration using the methods employed in the Capsitonin product of Bone Medical Limited. These may include the methods incorporated in Axcess formulations. More particularly, the active ingredient may be encapsulated in an enteric capsule capable of withstanding transit through the stomach. This may contain the active compound together with a hydrophilic aromatic alcohol absorption enhancer, for instance as described in WO02/028436. In a known manner the enteric coating may become permeable in a pH sensitive manner, e.g. at a pH of from 3 to 7. WO2004/091584 also describes suitable formulation methods using aromatic alcohol absorption enhancers.

Calcitonin or other family members may be formulated using the methods employed in the Unigene Enteripep® products. This may include methods as described in US5,912,014, US6,086,918 or US6,673,574. In particular, it may include the use of conjugation of the calcitonin or other family member to a membrane translocator such as the protein transduction domain of the HIV TAT protein, coformulation with one or more protease inhibitors, and/or a pH lowering agent and/or an acid resistant protective vehicle and/or an absorption enhancer which may be a surfactant.

Calcitonin or other family members may be formulated using the methods seen in the Oramed products, which may include formulation with omega-3 fatty acid as seen in WO2007/029238 or as described in US5,102,666.

Generally, the pharmaceutically acceptable salts (especially mono or di sodium salts), solvates (e.g. alcohol solvates) and hydrates of these carriers or delivery agents may be used.

Pharmaceutical compositions of the present invention typically contain a delivery effective amount of carrier such as 5-CNAC, i.e. an amount sufficient to deliver the calcitonin for the desired effect. Generally, the carrier such as 5-CNAC is present in an amount of 2.5% to 99.4% by weight, more preferably 25% to 50% by weight of the total composition. Oral administration of the pharmaceutical compositions according to the invention can be accomplished regularly, e.g. once or more on a daily or weekly basis; intermittently, e.g. irregularly during a day or week; or cyclically, e.g. regularly for a period of days or weeks followed by a period without administration. The dosage form of the pharmaceutical compositions of the instant invention can be any known form, e.g. liquid or solid dosage forms.

The liquid dosage forms include solution emulsions, suspensions, syrups and elixirs. In addition to the calcitonin and carrier such as 5-CNAC, the liquid formulations may also include inert excipients commonly used in the art such as, solubilizing agents e.g. ethanol; oils such as cottonseed, castor and sesame oils; wetting agents; emulsifying agents; suspending agents; sweeteners; flavorings; and solvents such as water. The solid dosage forms include capsules, soft-gel capsules, tablets, caplets, powders, granules or other solid oral dosage forms, all of which can be prepared by methods well known in the art. The pharmaceutical compositions may additionally comprise additives in amounts customarily employed including, but not limited to, a pH adjuster, a preservative, a flavorant, a taste-masking agent, a fragrance, a humectant, a tonicifier, a colorant, a surfactant, a plasticizer, a lubricant such as magnesium stearate, a flow aid, a compression aid, a solubilizer, an excipient, a diluent such as microcrystalline cellulose, e.g. Avicel PH 102 supplied by FMC corporation, or any combination thereof. Other additives may include phosphate buffer salts, citric acid, glycols, and other dispersing agents. The composition may also include one or more enzyme inhibitors, such as actinonin or epiactinonin and derivatives thereof; aprotinin, Trasylol and Bowman-Birk inhibitor. Further, a transport inhibitor, i.e. a [rho]-glycoprotein such as Ketoprofin, may be present in the compositions of the present invention. The solid pharmaceutical compositions of the instant invention can be prepared by conventional methods e.g. by blending a mixture of the calcitonin, the carrier such as 5-CNAC, and any other ingredients, kneading, and filling into capsules or, instead of filling into capsules, molding followed by further tableting or compression-molding to give tablets. In addition, a solid dispersion may be formed by known methods followed by further processing to form a tablet or capsule. Preferably, the ingredients in the pharmaceutical compositions of the instant invention are homogeneously or uniformly mixed throughout the solid dosage form.

Alternatively, the active compound may be formulated as a conjugate with said carrier, which may be an oligomer as described in US2003/0069170, e.g. 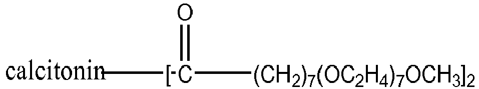 which when formed with salmon calcitonin is known as CT-025. Such conjugates may be administered in combination with a fatty acid and a bile salt as described there.

Conujugates with polyethylene glycol (PEG) may be used, as described for instance in Mansoor et al.

Alternatively, active compounds may be admixed with nitroso-N-acetyl-D,L-penicillamine (SNAP) and Carbopol solution or with taurocholate and Carbapol solution to form a mucoadhesive emulsion.

The active compound may be formulated by loading into chitosan nanocapsules as disclosed in Prego et al (optionally PEG modified as in Prego Prego C, Torres D, Fernandez-Megia E, Novoa-Carballal R, Quiñoá E, Alonso MJ.) or chitosan or PEG coated lipid nanoparticles as disclosed in Garcia-Fuentes et al. Chitosan nanoparticles for this purpose may be iminothiolane modified as described in Guggi et al. They may be formulated in water/oil/water emulsions as described in Dogru et al. The bioavailability of active compounds may be increased by the use of taurodeoxycholate or lauroyl carnitine as described in Sinko et al or in Song et al. Generally, suitable nanoparticles as carriers are discussed in de la Fuente et al and may be used in the invention.

Other suitable strategies for oral formulation include the use of a transient permeability enhancer (TPE) system as described in WO2005/094785 of Chiasma Ltd. TPE makes use of an oily suspension of solid hydrophilic particles in a hydrophobic medium to protect the drug molecule from inactivation by the hostile gastrointestinal (GI) environment and at the same time acts on the GI wall to induce permeation of its cargo drug molecules.

Further included is the use of glutathione or compounds containing numerous thiol groups as described in US2008/0200563 to inhibit the action of efflux pumps on the mucous membrane. Practical examples of such techniques are described also in Caliceti, P. Salmaso, S., Walker, G. and Bernkop-Schnürch, A. (2004) 'Development and in vivo evaluation of an oral insulin-PEG delivery system.' Eur. J. Pharm. Sci., 22, 315-323, in Guggi, D., Krauland, A.H., and Bernkop-Schnürch, A. (2003) 'Systemic peptide delivery via the stomach: in vivo evaluation of an oral dosage form for salmon calcitonin'. J. Control. Rel. 92,125-135, and in Bernkop-Schnürch, A., Pinter, Y., Guggi, D., Kahlbacher, H., Schöffmann, G., Schuh, M., Schmerold, I., Del Curto, M.D., D'Antonio, M., Esposito, P. and Huck, Ch. (2005) 'The use of thiolated polymers as carrier matrix in oral peptide delivery' - Proof of concept. J. Control. Release, 106, 26-33.

The active compound may be formulated in seamless microspheres as described in WO2004/084870 where the active pharmaceutical ingredient is solubilised as an emulsion, microemulsion or suspension, formulated into mini-spheres; and variably coated either by conventional or novel coating technologies. The result is an encapsulated drug in "pre-solubilised" form which when administered orally provides for predetermined instant or sustained release of the active drug to specific locations and at specific rates along the gastrointestinal tract. In essence, pre-solubilization of the drug enhances the predictability of its kinetic profile while simultaneously enhancing permeability and drug stability.

One may employ chitosan coated nanocapsules as described in US2009/0074824. The active molecule administered with this technology is protected inside the nanocapsules since they are stable against the action of the gastric fluid. In addition, the mucoadhesive properties of the system enhances the time of adhesion to the intestine walls (it has been verified that there is a delay in the gastrointestinal transit of these systems) facilitating a more effective absorption of the active molecule.

Methods developed by TSR1 Inc. may be used. These include Hydrophilic Solubilization Technology (HST) in which gelatin, a naturally derived collagen extract carrying both positive and negative charges, coats the particles of the active ingredient contained in lecithin micelles and prevents their aggregation or clumping. This results in an improved wettability of hydrophobic drug particles through polar interactions. In addition, the amphiphilic lecithin reduces surface tension between the dissolution fluid and the particle surface.

The active ingredient may be formulated with cucurbiturils as excipients.

Alternatively, one may employ the GIPET technology of Merrion Pharmaceuticals to produce enteric coated tablets containing the active ingredient with an absorption enhancer which may be a medium chain fatty acid or a medium chain fatty acid derivative as described in US2007/0238707 or a membrane translocating peptide as described in US7268214.

One may employ GIRES™ technology which consists of a controlled-release dosage form inside an inflatable pouch, which is placed in a drug capsule for oral administration. Upon dissolution of the capsule, a gas-generating system inflates the pouch in the stomach. In clinical trials the pouch has been shown to be retained in the stomach for 16-24 hours.

Alternatively, the active may be conjugated to a protective modifier that allows it to withstand enzymatic degradation in the stomach and facilitate its absorption. The active may be conjugated covalently with a monodisperse, short-chain methoxy polyethylene glycol glycolipids derivative that is crystallized and lyophilized into the dry active pharmaceutical ingredient after purification. Such methods are described in US5438040 and at www.biocon.com.

One may also employ a hepatic-directed vesicle (HDV) for active delivery. An HDV may consist of liposomes (≤150 nm diameter) encapsulating the active, which also contain a hepatocyte-targeting molecule in their lipid bilayer. The targeting molecule directs the delivery of the encapsulated active to the liver cells and therefore relatively minute amounts of active are required for effect. Such technology is described in US2009/0087479 and further at www.diasome.com.

The active may be incorporated into a composition containing additionally a substantially non-aqueous hydrophilic medium comprising an alcohol and a cosolvent, in association with a medium chain partial glyceride, optionally in admixture with a long-chain PEG species as described in US2002/0115592 in relation to insulin.

Alternatively, use may be made of intestinal patches as described in Shen Z, Mitragotri S, Pharm Res. 2002 Apr;19(4):391-5 'Intestinal patches for oral drug delivery'.

The active may be incorporated into an erodible matrix formed from a hydrogel blended with a hydrophobic polymer as described in US7189414.

Suitable oral dosage levels of calcitonin for adult humans to be treated may be in the range of 0.05 to 5mg, preferably about 0.1 to 2.5mg.

Dosages of calcitonin receptor agonists may be as described above for calcitonin, optionally scaled according to the relative agonist efficacy of the agonist compared to calcitonin itself in the test protocols described above.

The frequency of dosage treatment of patients may be from 1 to six times daily, for instance from two to four times daily. Treatment will desirably be maintained over a prolonged period of at least 6 weeks, preferably at least 6 months, preferably at least a year, and optionally for life.

Combination treatments for relevant conditions may be carried out using a composition according to the invention and separate administration of one or more other therapeutics. Alternatively, the composition according to the invention may incorporate one or more other therapeutics for combined administration.

Combination therapies according to the invention include combinations of an active compound as described with insulin, GLP-2, GLP-1, GIP, or amylin, or generally with other antidiabetics. Thus combination therapies including co-formulations may be made with insulin sensitizers including biguanides such as Metformin, Buformin and Phenformin, TZD's (PPAR) such as Pioglitazone, Rivoglitazone, Rosiglitazone and Troglitazone, dual PPAR agonists such as Aleglitazar, Muraglitazar and Tesaglitazar, or secretagogues including sulphonylureas such as Carbutamide, Chloropropamide, Gliclazide, Tolbutamide, Tolazamide, Glipizide, Glibenclamide, Glyburide, Gliquidone, Glyclopyramide and Glimepriride, Meglitinides/glinides (K+) such as Nateglinide, Repaglinide and Mitiglinide, GLP-1 analogs such as Exenatide, Liraglutide and Albiglutide, DPP-4 inhibitors such as Alogliptin, Linagliptin, Saxagliptin, Sitagliptin and Vildagliptin, insulin analogs or special formulations such as (fast acting) Insulin lispro, Insulin aspart, Insulin glulisine, (long acting) Insulin glargine, Insulin detemir), inhalable insulin - Exubra and NPH insulin, and others including alpha-glucosidase inhibitors such as Acarbose, Miglitol and Voglibose, amylin analogues such as Pramlintide, SGLT2 inhibitors such as Dapagliflozin, Remogliflozin and Sergliflozin as well as miscellaneous ones including Benfluorex and Tolrestat.

We hypothesise that the effect of enterally administered calcitonin family members, especially calcitonin itself, to improve glucose levels in patients suffering from insulin resistance may be explicable on the basis that calcitonin exerts opposite effects on skeletal muscle and on the liver in terms of elevating or reducing serum glucose respectively, and that enteral administration preferentially delivers calcitonin to the liver whereas parenteral administration favours its action on skeletal muscle. We suggest that when acting on skeletal muscle, calcitonin promotes consumption of glucose, so producing lactate which passes to the liver and signals the production by the liver of glucose. On the other hand, calcitonin acts in the liver to cause production of glycogen to store glucose.

This theory may explain the anomalous result reported in Lupulescu where very high injected doses of calcitonin resulted in a reduction of plasma glucose, in contrast to the results obtained in all the other reported animal studies. We suggest that the very high doses used may have been sufficient to activate the receptors for calcitonin in the liver, so overcoming the skeletal muscle response seen in other studies.

The active compound may be a calcitonin receptor agonist. Many of these are known in the art which are not peptides in nature but rather are synthetic small molecules. Other known calcitonin receptor agonists are calcitonin mimicking peptides. Examples of both types are discussed below.

Calcitonin receptor agonists for use according to the invention include those of JP2001294574 including those of the general formula 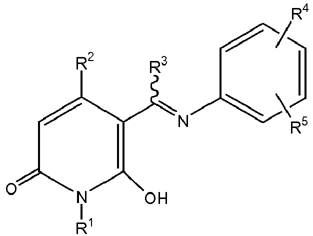 wherein R1 is H, hydroxyl group, a 1-4C alkyloxy group or a 7-10C aralkyloxy group; R2 and R3 are each a 1-4C alkyl group; and R4 and R5 are each H, a halogen atom, hydroxyl group, a 1-4C alkyl group, a 7-10C aralkyl group, a 1-4C alkyloxy group, a 7-10C aralkyloxy group, a 1-7C acyloxy group, amino group, a 1-4C alkylamino group, a 17-10C aralkylamino group, a 1-7C acylamino group, carboxyl group, a 1-4C alkyloxycarbonyl group, a 7-10C aralkyloxycarbonyl group, a carbamoyl group allowed to have at least one substituent, an acyl group or sulfamoyl group) and pharmacologically permissible salts thereof.

A preferred compound (SUN B8155) is one of the formula

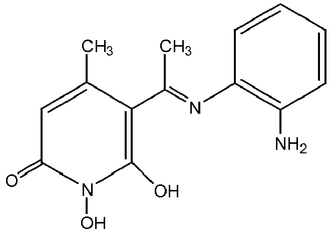

Calcitonin mimetics for use in accordance with the invention include those described in WO99/37604. Described there are compounds of the formula 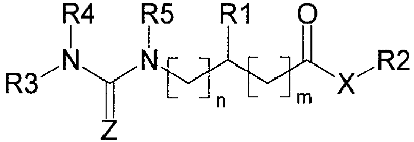 wherein
R1 and R2 are each members independently selected from the group consisting of hydrogen, alkyls having from 1 to 6 carbon atoms, alkenyls having from 1 to 6 carbon atoms, aryl, substituted aryl, alkylaryl, substituted alkylaryl, carbocyclic ring, substituted carbocyclic ring, heterocyclic ring, substituted heterocyclic ring, and combinations thereof, the combinations are fused or covalently linked and the substituents are selected from the group consisting of halogen, haloalkyl, hydroxy, aryloxy, benzyloxy, alkoxy, haloalkoxy, amino, monoalkylamino, dialkylamino, acyloxy, acyl, alkyl and aryl;
R3 is a 2,5 disubstituted aryl;
R4 and R5 are each independently selected from the group consisting of hydrogen and alkyls having from 1 to 6 carbon atoms, or taken together from a ring selected from the group consisting of saturated or unsaturated five-member rings, saturated or unsaturated six-member rings and saturated or unsaturated seven-member rings;
Z and X are each independently selected from the group NH, 0, S, or NR, wherein R is a lower alkyl group of from 1 to 6 carbon atoms; and
n and m are each independently an integer from 0 to 6.

These include compounds of the formula 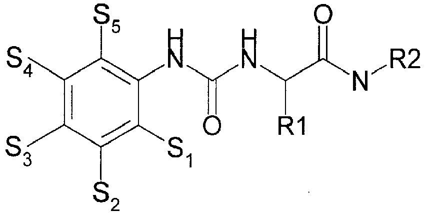 wherein,
R1 and R2 are each independently selected from the group consisting of hydrogen, alkyls having from 1 to 6 carbon atoms, alkenyls having from 1 to 6 carbon atoms, aryl, substituted aryl, alkylaryl, substituted alkylaryl, carbocyclic ring, substituted carbocyclic ring, heterocyclic ring, substituted heterocyclic ring, and combinations thereof, the combinations are fused or covalently linked and the substituents are selected from the group consisting of halogen, haloalkyl, hydroxy, aryloxy, benzyloxy, alkoxy, haloalkoxy, amino, monoalkylamino, dialkylamino, acyloxy, acyl, alkyl and aryl ;
S1, S3 and S4 are each independently selected from the group consisting of hydrogen, halogen, haloalkyl, hydroxy, aryloxy, benzyloxy, alkoxy, haloalkoxy, amino, monoalkylamino, dialkylamino, acyloxy, acyl, alkyl and aryl ; and S2 and S5 are each independently alkyl or aryl.

In particular, suitably R1 is 4-ethoxybenzyl, 1-ethyl-indolylmethyl, benzyl, 4-alloxybenzyl, 1-allyl-indolylmethyl, 4-chlorobenzyl, 4-flurobenzyl, 4-iodobenzyl, 2-naphthylmethyl or phenyl;
R2 is ethyl, allyl, benzyl or 2-naphthylmethyl;
and S2 and S5 are t-butyl.

Further examples of compounds which may be used are to be found in US7,396,936. These include compounds of the formula: 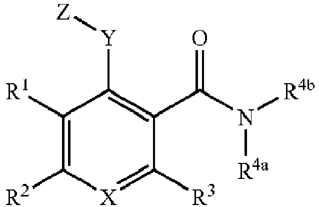 stereoisomers thereof, tautomers thereof, solvates thereof, prodrugs thereof, and pharmaceutically acceptable salts thereof; wherein
X is N or NO;
Y is a divalent substituted or unsubstituted aryl, heterocyclyl, or cycloalkyl group;
Z is -C(O)OR⁵ , -C(O)NR⁶R⁷, -NR⁶C(O)R⁵, -NR⁶C(O)NR⁶ R⁷, -C(O)R⁵ , -NR⁶ R⁷ , -OR⁸, -SO₂NR⁶ R⁷, -NR⁶SO₂R⁵, or -S(O)ₘR⁵;
R¹ is -H, -C(O)OR⁹, -C(O)NR¹⁰R¹¹, -CN, -C(O)R⁹, or -NR¹⁰C(O)R⁹ ;
R² is -(C₁₋₂ alkyl) -R¹², wherein the C₁₋₂ alkyl is substituted or unsubstituted;
R³ is a C₂₋₆ branched or unbranched alkyl group, optionally substituted with one or more F;
R^{4a} and R^{4b} are each independently -H or substituted or unsubstituted C₁₋₄ alkyl group;
R⁵ is a substituted or unsubstituted aralkyl, heteroaralkyl, heterocyclylalkyl, or cycloalkyl alkyl group;
R⁶ and R⁷ are each independently -H or a substituted or unsubstituted aralkyl, heteroaralkyl, heterocyclylalkyl, or cycloalkyl alkyl group; or R⁶ and R⁷, when attached to the same atom, together form a substituted or unsubstituted heterocyclyl group;
R⁸ is a substituted or unsubstituted, branched or unbranched C₂₋₆ alkyl, C₂₋₆ alkenyl, C₂₋₆ alkynyl, or C₇₋₁₀ aralkyl group; R⁹ is -H or a substituted or unsubstituted alkyl, heteroalkyl, alkenyl, alkynyl, aryl, aralkyl, heterocyclyl, or heterocyclylalkyl group;
R¹⁰ and R¹¹are each independently -H or a substituted or unsubstituted alkyl, heteroalkyl, alkenyl, alkynyl, aryl, aralkyl, heterocyclyl, or heterocyclylalkyl group; or R¹⁰ and R¹¹, when attached to the same atom, together form a substituted or unsubstituted heterocyclyl group;
R¹² is a substituted or unsubstituted cycloalkyl, aryl, heteroaryl group, or is an unsubstituted alkyl group; and m is 0, 1 or 2.

Examples include 5-Carbamoyl-2-[2-(4-fluoro-phenyl)-ethyl]-4-{4-[(furan-2-ylmethyl)-carbamoyl]-phenyl}-6-propyl-nicotinic acid ethyl ester;
5-Carbamoyl-2-(2-cyclohexyl-ethyl)-4-{4-[(furan-2-ylmethyl)-carbamoyl]-phenyl}-6-isobutyl-nicotinic acid ethyl ester; 5-Carbamoyl-2-[2-(4-fluoro-phenyl)-ethyl]-4-{4-[(furan-2-ylmethyl)-carbamoyl]-phenyl}-6-isobutyl-nicotinic acid ethyl ester;
5-Carbamoyl-2-(2-cyclohexyl-ethyl)-4-{4-[(furan-2-ylmethyl)-carbamoyl]-phenyl}-6-propyl-nicotinic acid ethyl ester; 5-Carbamoyl-2-[2-(4-fluoro-phenyl)-ethyl]-6-propyl-4-{4-[(pyridin-3-ylmethyl)-carbamoyl]-phenyl}-nicotinic acid ethyl ester;
5-Carbamoyl-2-(2-cyclohexyl-ethyl)-6-propyl-4-{4-[(pyridin-3-ylmethyl)-carbamoyl]-phenyl}-nicotinic acid ethyl ester; 2-[2-(4-Fluoro-phenyl)-ethyl]-4-{4-[(furan-2-ylmethyl)-carbamoyl]-phenyl}-6-isobutyl-pyridine-3,5-dicarboxylic acid diamide;
5-Carbamoyl-6-ethyl-2-[2-(4-fluoro-phenyl)-ethyl]-4-{4-[(furan-2-ylmethyl)-carbamoyl]-phenyl}-nicotinic acid ethyl ester; and
5-Carbamoyl-2-[2-(4-fluoro-phenyl)-ethyl]-6-isobutyl-4-{4-[(pyridin-3-ylmethyl)-carbamoyl]-phenyl}-nicotinic acid ethyl ester.

Additionally, the active compound may be as described in WO98/37077. Hence it may be of the formula 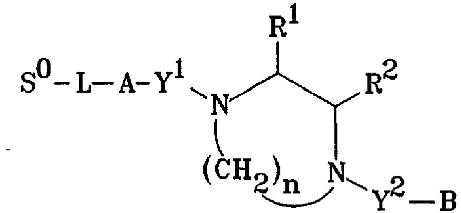 wherein
A and B are each members independently selected from the group consisting of aryl, substituted aryl, carbocyclic ring, substituted carbocyclic ring,
heterocyclic ring, substituted heterocyclic ring, and combinations thereof, said
combinations being fused or covalently linked and said substituents being selected from the group consisting of halogen, haloalkyl, hydroxy, aryloxy, benzyloxy, alkoxy, haloalkoxy, amino, monoalkylamino, dialkylamino, acyloxy, acyl, alkyl and aryl; R¹ and R² are each independently selected from the group consisting of hydrogen and alkyl groups having from 1 to 6 carbon atoms, or taken together form a ring selected from the group consisting of saturated or unsaturated five member rings, saturated or unsaturated six member rings and saturated or unsaturated seven-member rings; Y¹ and Y² are each independently a bond or a divalent radical selected from the group consisting of -CH2-, -NHC(O)-, -NRC(O)-, -NHC(S)-, -NRC(S)-, -NHC(=NH)-, -OC(O)-, -C(O)-, and -C(S)-, in which R is a lower alkyl group of from one to six carbon atoms; and n is an integer of from zero to four.

Suitable examples include

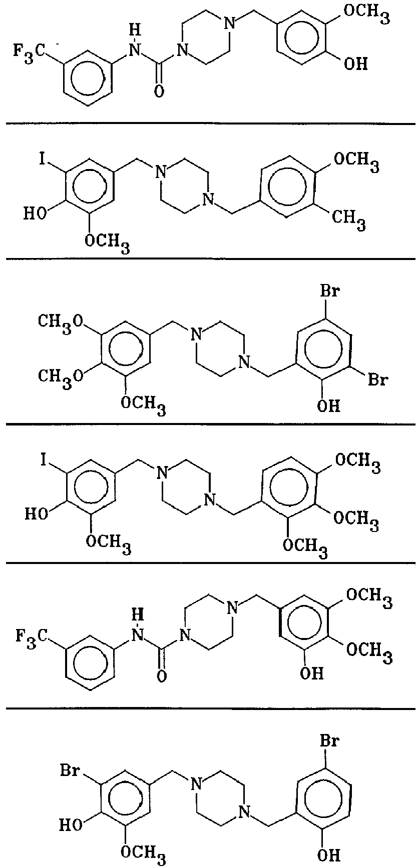

Amongst peptide based mimetics that may be employed are those of US5,698,521, including either of
Acetyl-Trp-Xaa1 -Gln-Xaa2 -Ile-Thr-Xaa3 -Leu-Xaa4 -Pro-Gln-Xaa5 -Pro-Xaa6 -Xaa7 -Phe-Gly-COOH and
Acetyl-Trp-Xaa1 -Gln-Xaa2 -Ile-Thr-Xaa3 -Leu-Xaa4 -Pro-Gln-Xaa5-Pro-Xaa6 -Xaa7 -Phe-COOH (SEQ ID NO.2 ); wherein Acetyl is CH3 CO--, Xaa1 is isovaline, Xaa2,3,4,5, and 6 are 2-aminoisobutyric acid and Xaa7 is 4-methyl proline.

The invention will be further described and illustrated by the following examples in which reference is made to the accompanying drawings, in which:
Figure 1 shows weight changes during the study in all treatment groups; a) - vehicle and rosiglitazone groups; b) calcitonin and 5-CNAC treated groups;
Figure 2 shows (in panels a and d) results of oral glucose tolerance test (OGTT) over 120 minutes for a) vehicle and rosiglitazone treated groups and d) calcitonin and 5-CNAC treatment groups; and (in panels b and e) relative change in glucose levels during the 120 min OGTT normalized to t=0 for b) vehicle and rosiglitazone treated groups and e) calcitonin and 5-CNAC treatment groups. The bar graphs (in panels c and f) show the integrated AUC;
Figure 3 shows (in panels a and d) total insulin levels measured in DIO rats in treatment groups a) vehicle and rosiglitazones and d) 5-CNAC and calcitonin, during the 120 min of OGTT; and (in panels b and e) change in insulin levels in DIO rats from t=0 in treatment groups b) vehicle and rosiglitazones and e) 5-CNAC and calcitonin during the 120 min of OGTT. The bar graphs (in panels c and f)show the integrated AUC;
Figure 4 shows results of OGTT in healthy control animals treated with calcitonin and 5-CNAC. a) Total glucose levels monitored in the 5-CNAC and calcitonin treated groups. b) Relative change in glucose levels monitored during the 120 min OGTT in the 5-CNAC and calcitonin treated groups, normalized to t=0. c) integrated AUC;
Figure 5 shows insulin levels during the OGTT of Figure 4. a) Total insulin levels monitored in the 5-CNAC and calcitonin treated groups. b) Relative change in insulin levels monitored during the 120 min OGTT in the 5-CNAC and calcitonin treated groups, normalized to t=0. c) integrated AUC;
Figure 6 shows the amino acid sequences of several amylin like peptides compared to amylin (SEQ ID NOs 1,2,6,10,48-56);
Figure 7 shows the amino acid sequences of several amylin receptor agonists compared to amylin (SEQ ID NOs:39-47);
Figure 8 shows results obtained in Example 4 showing the effect of oral salmon calcitonin on weight gain in normal rats during an 8-week treatment period. Data are plotted as mean+/-SEM;
Figure 9 shows results obtained in Example 4 showing the effect of oral salmon calcitonin partially protecting against OVX-induced weight gain during a 7-week treatment period. Data are plotted+/-SEM; and
Figure 10 shows results obtained in Example 4 showing that oral salmon calcitonin dosed once daily does not protect against OVX-induced weight gain during the 8-week treatment period. Data are plotted+/-SEM and starting weights are normalised.
Figure 11, in panels A to D shows data produced in Example 5 demonstrating the oral bioavailability of sCT formulated with SNAC.
Figure 12, further illustrates the data produced in Example 5 demonstrating the oral bioavailability of sCT formulated with SNAC.

### Example 1: Effect of salmon calcitonin in combination with 5-CNAC on response to an oral glucose tolerance test in DIO rats in comparison to Rosiglitazone

### MATERIALS AND METHODS.

### Animals

A total of 48 selectively bred male DIO rats were used. At the beginning of the experiment, the animals had reached an age of 35 weeks, out of which 31 weeks were on high-fat diet. Rosiglitazone was suspended in 10% Hydroxypropyl beta-cyclodexetrin (Cat.no. A0367.0100. Salmon calcitonin (CT) and 5-CNAC (N-(5-chlorosalicyloyl)-8-aminocaprylic acid) were suspended in MilliQ water. The animals were treated with once daily doses of either 5-CNAC 150mg/kg/day or that amount of 5-CNAC in combination with Calcitonin 2mg/kg/day for 5 weeks or with 3mg/kg or 10 mg/kg of Rosiglitazone (a known anti-diabetic, used as a positive control).

At day -7, 21 and 42 fasted serum and urine samples were collected and whole body tissue composition was evaluated by MR scanning. At day 42, an oral glucose tolerance test (OGTT) was performed to evaluate glucose homeostasis in the rats. At day -1 the animals were stratified according to weight and whole body fat mass (assessed by a 4-in-1 EchoMRI scanner) to the different treatment groups. The groups were randomized in three teams by body weight.

First day of dosing was day 0. Animals were dosed per oral 5 ml/kg. The compound solution was administered once daily at 7:00 AM - 2:00 PM during the whole study (0-42 days) per oral gavage using a gastric tube connected to a 5 ml syringe (luer lock^{™}. Becton).

### Weight gain

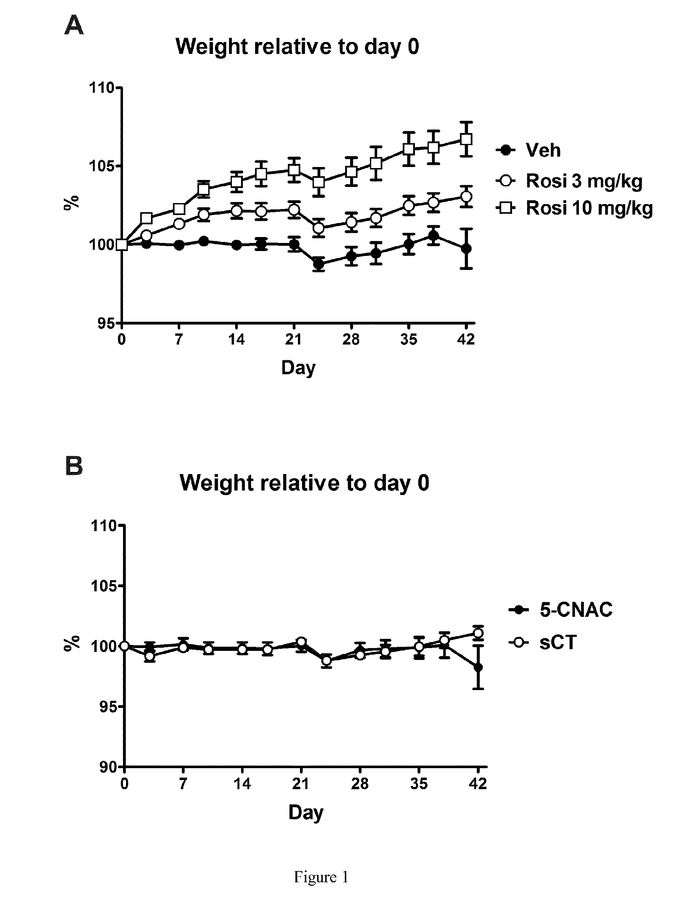

Since glitazones are known to induce weight gain, the animals were continuously weighed and as seen in Figure 1.a and 1.b animals in the vehicle group kept a steady body weight throughout the study. In contrast, rats treated with 3 mg/kg Rosiglitazone displayed a significant (p=0.05) increase in their body weight during the course of the study, starting immediately after treatment initiation. Animals treated with 10 mg/kg Rosiglitazone also had significantly increased body weight compared to that of the animals receiving only vehicle (p=0.0001). Neither the CT treated group nor the 5-CNAC group display any change in their body weight (p=0.8).

To assess the distribution of the gained weight during the treatment program the animals were subjected to MR analysis after termination. Two parameters were investigated: 1) change in fatty mass and, 2) change in total water content.

Both rosiglitazone doses led to significant increases (p<0.0001) in total fat, compared to the rats in the vehicle group, as well as in the 5-CNAC group. However, the CT treated group had significantly lower increase in the fat content compared to rosiglitazone group (p<0.0001)(see table 2), but a significant increase compared to the 5-CNAC and vehicle groups. Neither of the treatments appeared to lead to accumulation of water. Monitoring of food intake showed that the increased bodyweight was not caused by increased food intake (data not shown).

### ORAL GLUCOSE TOLERANCE TEST (OGTT)

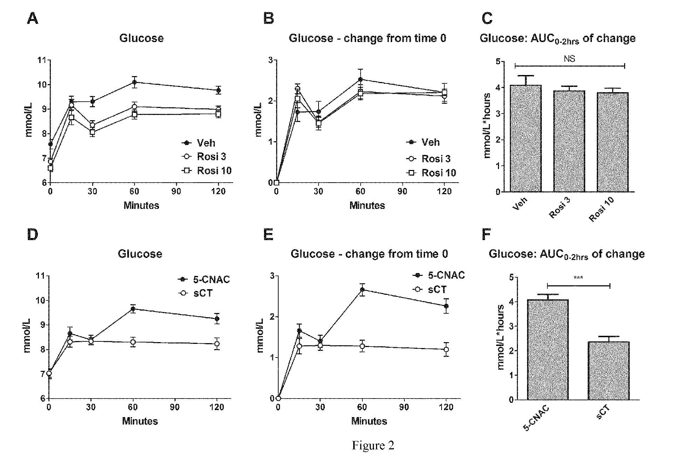

In order to test the glucose handling in different treatment groups, an oral glucose tolerance test (OGTT) was performed. Baseline plasma glucose levels of animals receiving only vehicle were significantly higher than those of the Rosiglitazone treatment groups (ANOVA p=0.0005; Dunnett adjusted: Vehicle vs. Rosiglitazone 3 mg/kg p=0.006; Vehicle vs. Rosiglitazone 10 mg/kg p=0.0003), showing that Rosiglitazone indeed reduces basal glucose levels (Figure 2a). During the OGTT, plasma glucose levels in the vehicle group increased up to 10.1 mmol/L glucose and remained elevated during the whole OGTT period, establishing a state of hyperglycaemia, as expected for DIO rats displaying with defective glucose clearance as one of the model characteristics. In contrast, animals receiving Rosiglitazone treatment displayed better glucose regulation at baseline and during the OGTT (Figure 2a & 2b), as expected. When comparing CT treated animals to the 5CNAC group, CT strongly reduced peak glucose levels (Figure 2c & 2d). In the CT group the blood glucose levels increased to a maximum of 8.2 mM 15 minutes after OGTT, a level that persisted during the OGTT, whereas in the 5-CNAC group the glucose level reached peak levels of 9.8mM at 60 minutes, and the increase persisted throughout the OGTT.

The area under the curve (AUC) of the net change in plasma glucose clearance was used to compare the effects of the different treatment strategies. Rosiglitazone and CT treatment both caused significantly increased glucose clearance during the OGTT. Treatment with 10 mg/kg of Rosiglitazone displayed significantly faster glucose clearance compared to that of the effect of 3 mg/kg of Rosiglitazone. The effect of CT on the AUC of change was markedly larger than the changes seen in the Rosiglitazone groups.

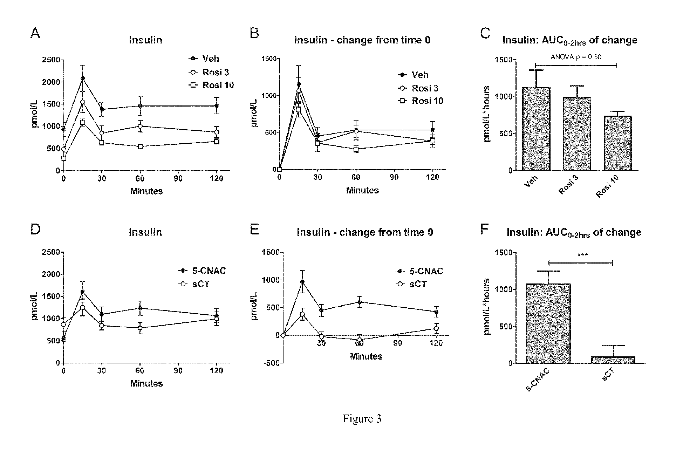

Plasma insulin response curves were calculated for all animals in different treatment groups, reflecting their plasma insulin levels at different time points during the OGTT. The vehicle group for Rosiglitazone had higher basal insulin levels of app. 5.34 µg/L and an exaggerated insulin response peaking at 11.97 µg/L 15 minutes after the infusion of glucose. After the initial response, insulin levels decreased to app. 8.40 µg/L and this hyperinsulinaemic state remained unchanged during the rest of the OGTT (Figure 3). Rosiglitazone dose-dependently reduced both basal and OGTT induced insulin levels (Figure 3a &b) when compared to the vehicle group. CT treatment led to an attenuated insulin response when compared to the 5-CNAC group, with peak insulin levels observed 15 minutes after the glucose infusion, hereafter insulin levels return to basal levels of 4.53 µg/L, whereas the 5-CNAC treated group also peak after 15 minutes, but the levels stay in the hyperinsulinaemic state throughout the OGTT.

The above results are further reflected in Figure 3.e illustrating the AUC of change, where CT treatment leads to the lowest change in insulin levels, an effect which is markedly larger than that of Rosiglitazone.

### Example 2: Effect of salmon calcitonin in combination with 5-CNAC on response to an oral glucose tolerance test in Sprague Dawley rats

### OGTT IN HEALTHY RATS

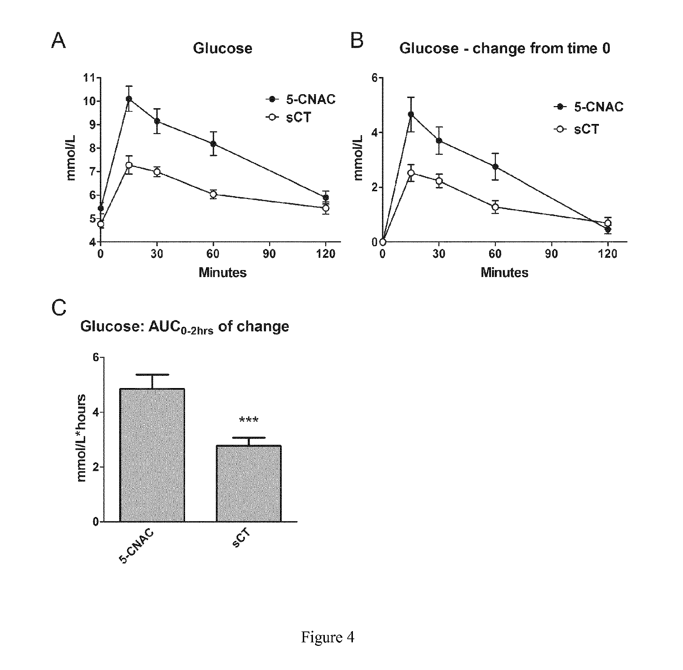

To further investigate whether CT possesses glucose lowering properties, we performed an OGTT on 20 (age and sex-matched) control lean Sprague Dawley rats, 10 of which were assigned to the 5-CNAC group and 10 to the CT treatment group. The rats were treated bi daily for 5 weeks using the same dose as in the DIO rats. CT treatment lowered basal glucose levels in these animals (Figure 4a). During OGTT the vehicle group showed a peak glucose level of 10.1 mmol/l after 15 minutes, and then the glucose levels returned to near-baseline. Treatment with CT prevented this drastic peak, and kept the glucose levels lower leading to faster clearance, displaying a beneficial effect on the glycaemic control and blood glucose clearance in the CT treated group. Notably, both vehicle and CT treated animals reached basal blood glucose levels at the end of experiment t=120 min. AUC calculations of the change during OGTT confirmed that CT treatment potently reduces glucose levels (Figure 4c).

Furthermore, we also monitored the effects of CT on insulin levels during the OGTT. During the OGTT, the vehicle treated group responded by increasing the insulin levels up to 2.5 µg/L, a level that persisted for approximately 30 minutes. After 120 minutes, the insulin levels in vehicle treated group returned to basal. The CT treated animals, responded to the OGTT by a small increase of approximately 0.5 µg/L, and the insulin level returned to basal after only 60 min. The change in the insulin levels of vehicle treated animals compared to controls from time 0 until 120 minutes is not significant (p=0.53), whereas the AUC of change is significant between the groups (p=0.08). Results are presented in Figures 5a and 5b.

### Example 3: Screening for calcitonin receptor agonists.

### CALCITONIN AND AMYLIN RECEPTOR AGONIST ASSAYS

To identify calcitonin receptor (CTR) and amylin receptor agonist we used COS-7 cells transfected with the CTR in the absence or presence of the Receptor amplifying proteins (RAMPs). The CTR is a G-protein coupled receptor (GPCR), and many opportunities to assay agonists of GPCRs are available (1-12), of which induction of cAMP is an potential assay candidate. COS-7 cells were chosen for due to lack of phenotypically significant levels of endogenous RAMPs, CT receptors, and CL (13-15). Without significant background expression of such receptor components, defined receptor subtypes can be accurately compared.

We generated 4 assays.
1. An assay that was specific for the CTR, by over expressing the CTR in the absence of RAMP1, RAMP2 or RAMP3.
2. An assay that was specific for activating the Amylin receptor by over expressing the CTR with RAMP1 but in the absence of RAMP2 or RAMP3
3. An assay that was specific for activating the Amylin receptor by over expressing the CTR with RAMP2 but in the absence of RAMP1 or RAMP3
4. An assay that was specific for activating the Amylin receptor by over expressing the CTR with RAMP3 but in the absence of RAMP1 or RAMP2.

The compounds were considered specific inducers of a specific receptor by a significant induction of cAMP of more than 50%, compared to vehicle control (ether in the presence of absence of 1mM IBMX).

The calcitonin resorption sequence and amylin receptor sequence is already known (16;16-35), in various species, in different splice variants.

To assay receptor agonism, wells were gown to 80% confluence in 75 cm2 flasks. Cells were cotransfected with 300 ng of pcDNA-CTR construct and Imicrogram of either pcDNA-RAMP-1, pcDNA-RAMP-2 or pcDNA-RAMP-3 using 7.8 µL of FuGene 6 reagent. After 48 hours cells were lifted by trypsin treatment, and centrifuged at 500 x g, and resuspended in cyclase buffer (DMEM containing 0.1% (wt/vol) BSA and 1 mM IBMX). Cells (5 x 105) were aliquoted into 1.5 ml Eppendorf tubes and preincubated for 20 min at 37 C. Cells were subsequently incubated for 18 min in the absence (basal) or presence of increasing concentrations of agonists. The peptide. Forskolin was included to determine maximal cAMP accumulation for this system, as positive control.

Following incubation, the reactions were centrifuged in a Beckman microcentrifuge at 12,000 x g for 1 min at 4 C. The cells were washed with PBS and recentrifuged. The cAMP was extracted with 0.5 ml absolute ethanol. The samples were evaporated to dryness and reconstituted in buffer (50 mM sodium acetate, 1 mM theophylline). Levels of cAMP were assayed using a specific assay for detection for cAMP.. e.g Quantification of the intracellular second messenger cAMP was performed according to the EIA kit protocol (Amersham Biosciences, US) alternately an RIA approach following acetylation of samples was followed. Unbound radioactivity was extracted for 15 min at 4 C with 1 ml separation buffer [100 mM dipotassium hydrogen phosphate, 100 mM potassium phosphate, pH 7.4, containing 0.25% (wt/vol) BSA and 0.2% (wt/vol) charcoal and separated from antibody-bound radioactivity by centrifugation for 15 min at 4,000 x g. The supernatant was aspirated, and pellets were counted on a Packard γ-counter.

Alternative assay techniques for the identification of GPCR agonists have been extensive descried in the literature, recently including (1-12), and can be performed by an expert in the field.

### Example 4: Oral salmon calcitonin reduces weight gain in both healthy Sprague-Dawley rats and Sprague-Dawley rats with ovariectomy-induced weight gain.

A total of 40 rats were divided into 4 groups, two bilateral ovariectomy (OVX) groups and two normal groups. The animals in both the normal and the OVX groups were treated with either bidaily doses of either 5-CNAC 150mg/kg/day or that amount of 5-CNAC in combination with salmon calcitonin 2mg/kg/day for 7 or 8 weeks during which their weight was monitored once a week.

The first day of dosing was day 0. Animals were dosed per oral 5 ml/kg. The compound solution was administered twice daily, the first dose at 7:00 AM and the second dose 8 hous later (between 3 and 4PM) during the whole study. The dosing was done by oral gavage using a gastric tube connected to a 5 ml syringe. Feeding was ad libitum.

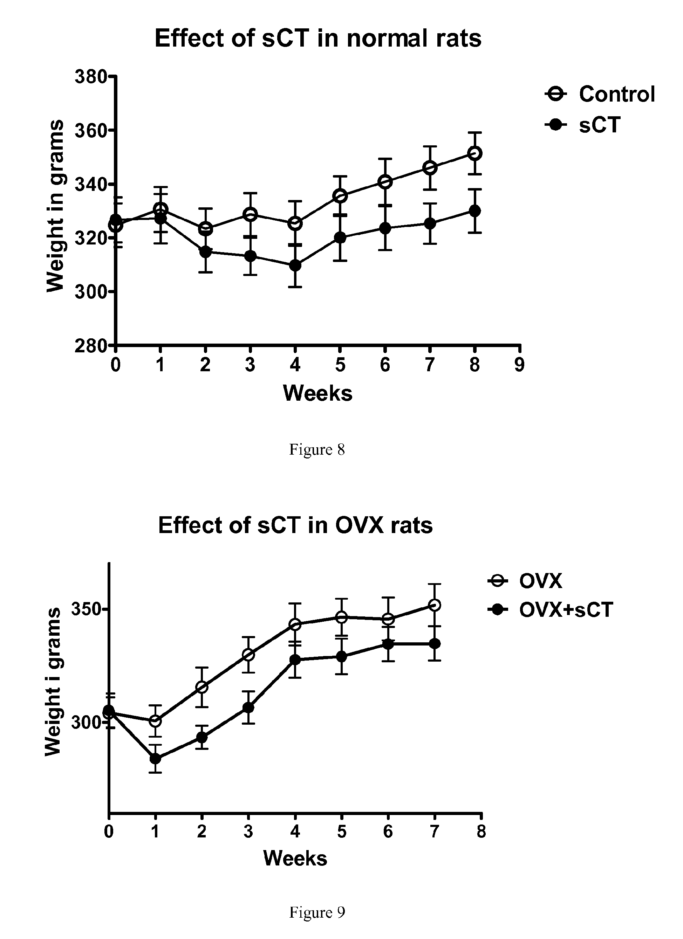

As seen in Figure 8 treatment of normal rats twice daily with oral salmon calcitonin led to a reduction of the natural weight gain observed in the control group during the treatment period. Furthermore, as seen in Figure 9, in the OVX groups oral salmon calcitonin treatment protected partially against the OVX-induced increase in bodyweight. Thus, oral salmon calcitonin treatment when dose twice daily leads to a reduction in weight gain, an effect likely mediated via regulation appetite.

By comparison a similar study in which the dosing was once per day showed no reduction in weight in comparison to controls. A total of 20 rats were bilaterally ovariectomized (OVX). The animals were treated once daily with either 5-CNAC 150mg/kg/day alone or that amount of 5-CNAC in combination with Calcitonin 2mg/kg/day for 8 weeks during which the weight was monitored once a week. Feeding was again ad libitum.

The first day of dosing was day 0. Animals were dosed per oral 5 ml/kg. The compound solution was administered once daily between 7:00 and 8:00 AM during the whole study per oral gavage using a gastric tube connected to a 5 ml syringe.

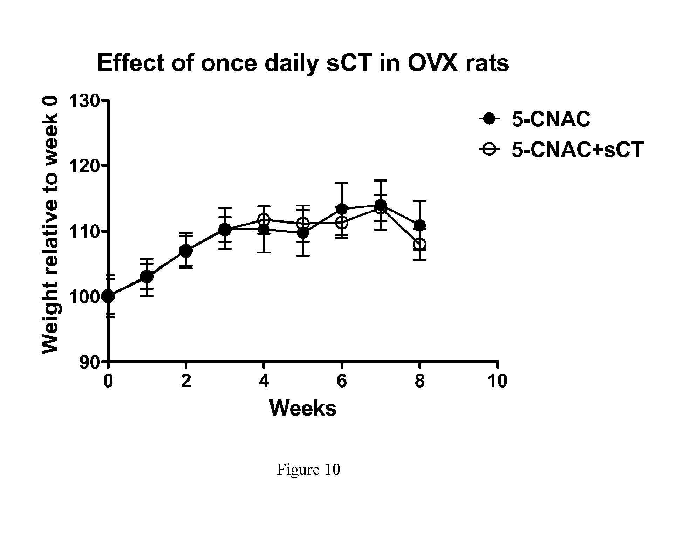

As seen in figure 10, no effect on OVX-induced weight gain was observed when the animals were dosed once daily, corresponding well to the data from Example 1 showing no weight gain in the DIO rats, when they were treated once daily.

### Example 5: Salmon calcitonin formulated with SNAC is bioavailable as demonstrated by lowering of Type II collagen resorption

To test whether observed effects of oral sCT were specifically dependent on an oral formulation with the carrier 5-CNAC, a comparison study, with the two oral carriers 5-CNAC and SNAC, was conducted. Here, lowering of cartilage resorption as measured by CTX-II is used as a surrogate marker for oral bioavailability and hence effectiveness in blood sugar control.

Fasted rats were treated with oral doses of carrier alone (5-CNAC or SNAC), sCT alone or sCT in combination with carrier (5-CNAC or SNAC). Blood sampling was conducted at baseline, one, three and six hours after oral dosing, and serum was isolated. Concentrations of CTX-II were subsequently measured in the serum samples.

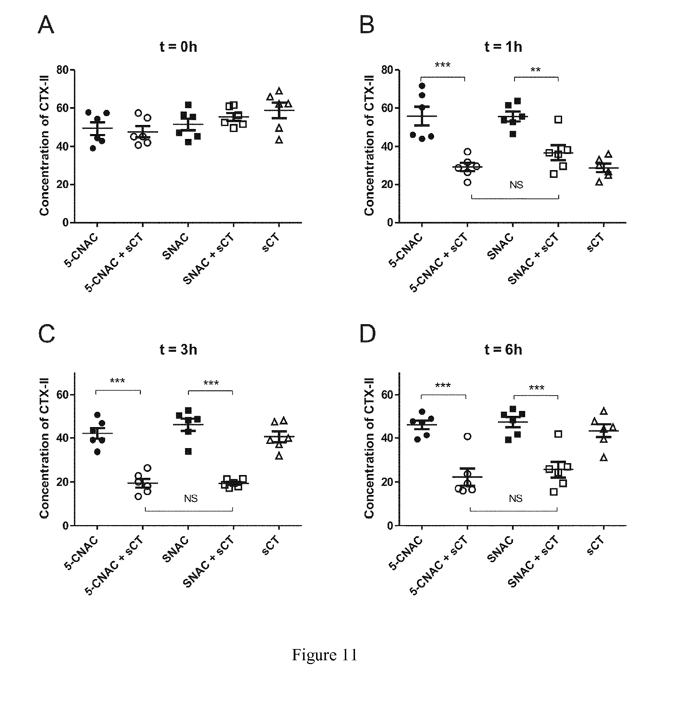

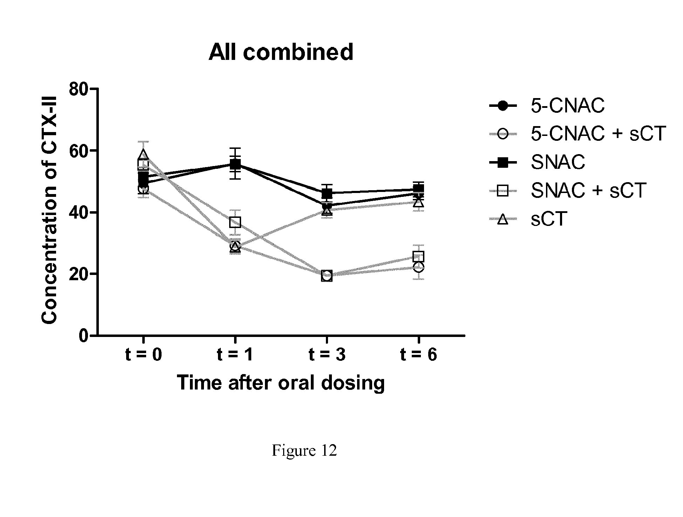

Rats were randomized according to weight into five groups and fasted over night. The rats were given oral doses of carrier alone (150mg/kg) (5-CNAC or SNAC), salmon calcitonin alone (2mg/kg) or carrier (150mg/kg) and salmon calcitonin (2mg/kg) together. Blood samples were taken from tail vein at baseline, one, three and six hours after oral dosing. Serum was isolated from the blood samples and CTX-II concentrations were measured in undiluted serum samples. Figure 11, panels A-D shows CTX-II concentrations at the indicated time points. Figure 12 shows combined results for all treatment groups over time. Each group contained 6 animals. Statistical analyses were conducted with one-way ANOVA and Bonferroni's Multiple Comparison Test to adjust for multiple tests. Error bars indicate standard error of mean (SEM). Asterisks indicate: * * = p < 0.01, * * * = p < 0.001.

It was found that one hour after oral dosing, sCT alone, sCT with 5-CNAC and sCT with SNAC was all significantly lowered the CTX-II concentration in comparison to the two groups with carrier alone (Figure 11, panel B). At three and six hours after oral dosing it was found that sCT given alone caused the CTX-II concentrations to return to the baseline level (Figure 11, panels C and D and Figure 12). In contrast, when sCT was given together with either 5-CNAC or SNAC, a continued reduction in CTX-II concentrations was observed at both three and six hours after oral dosing (Figure 11, panels C and D and Figure 12).

An important observation is that CTX-II levels are lowered equally well by both sCT with 5-CNAC and sCT with SNAC, and no significant differences is observed between the two groups at any of the indicated time points. These results imply that the reported effects of sCT is not dependent on 5-CNAC specifically. It may be concluded that significant long lasting effects of oral sCT are dependent on an oral formulation containing a carrier. However, as seen in Figures 11 and 12, two different oral carriers (5-CNAC and SNAC) in combination with sCT give equivalent results. This signifies that the observed effects of sCT are not restricted to one specific oral formulation and this finding may be expected to extend to the blood sugar lowering effects of sCT.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. All prior teachings acknowledged above are hereby incorporated by reference. No acknowledgement of any prior published document herein should be taken to be an admission or representation that the teaching thereof was common general knowledge in Australia or elsewhere at the date hereof.

### References:

Albrandt K, Mull E, Brady EM, Herich J, Moore CX, Beaumont K. Molecular cloning of two receptors from rat brain with high affinity for salmon calcitonin. FEBS Lett 1993; 325(3):225-32.
Aldred J.P., Clements G.R., Schlueter R.J., Bastian J.W., Dailey J.P. and Wazeteer F.X.; Symposium on Thyrocalcitonin and C-cells, ed by S. Taylor, pp. 379-389, W. Heinemann, London, 1968
Arvinte T, Drake AF. Comparative study of human and salmon calcitonin secondary structure in solutions with low dielectric constants. J Biol Chem 1993; 268(9):6408-14.
Bello NT, Kemm MH, Moran TH. Am J Physiol Regul Integr Comp Physiol. 2008 Jul;295(1):R76-81. Epub 2008 May 14.
Bernkop-Schnürch, A., Pinter, Y., Guggi, D., Kahlbacher, H., Schöffmann, G., Schuh, M., Schmerold, I., Del Curto, M.D., D'Antonio, M., Esposito, P. and Huck, Ch. (2005) The use of thiolated polymers as carrier matrix in oral peptide delivery - Proof of concept. J. Control. Release, 106, 26-33.
Beukers MW, Ijzerman AP. Techniques: how to boost GPCR mutagenesis studies using yeast. Trends Pharmacol Sci 2005; 26(10):533-9.
Blahos J, Svoboda Z, Höschl C. Endokrinologie. 1976;68(2):226-30.
Caliceti, P. Salmaso, S., Walker, G. and Bernkop-Schnürch, A. (2004) Development and in vivo evaluation of an oral insulin-PEG delivery system. Eur. J. Pharm. Sci., 22, 315-323.
Chelikani PK, Haver AC, Reidelberger RD. Am J Physiol Regul Integr Comp Physiol. 2007 Nov;293(5):R1798-808. Epub 2007 Aug 29.
Cornish J, Reid IR. Effects of amylin and adrenomedullin on the skeleton. J Musculoskelet Neuronal Interact 2001; 2(1):15-24.
Cornish J, Callon KE, Bava U, Kamona SA, Cooper GJ, Reid IR. Effects of calcitonin, amylin, and calcitonin gene-related peptide on osteoclast development. Bone 2001; 29(2):162-8.
Dacquin R, Davey RA, Laplace C, Levasseur R, Morris HA, Goldring SR et al. Amylin inhibits bone resorption while the calcitonin receptor controls bone formation in vivo. J Cell Biol 2004; 164(4):509-14.
de la Fuente M, Csaba N, Garcia-Fuentes M, Alonso MJ. Nanomed. 2008 Dec;3(6):845-57.
Dogru ST, Calis S, Oner F. J Clin Pharm Ther. 2000 Dec;25(6):435-43
Eglen RM. Assessing GPCR activation using protein complementation: a novel technique for HTS. Biochem Soc Trans 2007; 35(Pt 4):746-8.
Garcia-Fuentes M, Prego C, Torres D, Alonso MJ. Eur J Pharm Sci. 2005 May;25(1):133-43.
Garcia-Fuentes M, Torres D, Alonso MJ Int J Pharm. 2005 May 30;296(1-2):122-32. Epub 2005 Apr 7.
Gattereau A, Bielmann P, Durivage J, Davignon J, Larochelle P. J Clin Endocrinol Metab. 1980 Aug;51(2):354-7. Effect of acute and chronic administration of calcitonin on serum glucose in patients with Paget's disease of bone.
Giugliano D, Passariello N, Sgambato S, D'Onofrio F. Lancet. 1980 Mar 22;1(8169):653.
Giugliano D, Passariello N, Sgambato S, Torella R., Ceriello A. and D'Onofrio F., Diabete & Metabolisme (Paris) 1982, 8, 213-216.
Giustina G, Cerudelli B, Cimino A, Rigosa C, Rotondi A, Radaeli E. J Endocrinol Invest. 1985 Feb;8(1):19-23.
Greeley GH Jr, Cooper CW, Jeng YJ, Eldridge JC, Thompson JC., Regul Pept. 1989 Mar;24(3):259-68.
Guggi D, Kast CE, Bernkop-Schnürch A. Pharm Res. 2003 Dec;20(12):1989-94.
Guggi, D., Krauland, A.H., and Bernkop-Schnürch, A. (2003) Systemic peptide delivery via the stomach: in vivo evaluation of an oral dosage form for salmon calcitonin. J. Control. Rel. 92, 125-135.
Hay DL, Howitt SG, Conner AC, Doods H, Schindler M, Poyner DR. A comparison of the actions of BIBN4096BS and CGRP(8-37) on CGRP and adrenomedullin receptors expressed on SK-N-MC, L6, Col 29 and Rat 2 cells. Br J Pharmacol 2002; 137(1):80-6.
Hay DL, Howitt SG, Conner AC, Schindler M, Smith DM, Poyner DR. CL/RAMP2 and CL/RAMP3 produce pharmacologically distinct adrenomedullin receptors: a comparison of effects of adrenomedullin22-52, CGRP8-37 and BIBN4096BS. Br J Pharmacol 2003; 140(3):477-86.
Hay DL, Poyner DR, Smith DM. Desensitisation of adrenomedullin and CGRP receptors. Regul Pept 2003; 112(1-3):139-45.
Hay DL, Poyner D, Dickerson I. CGRP receptor heterogeneity: a role for receptor component protein? Trends Endocrinol Metab 2003; 14(1):3-4.
Hay DL, Christopoulos G, Christopoulos A, Sexton PM. Determinants of 1-piperidinecarboxamide, N-[2-[[5-amino-1-[[4-(4-pyridinyl)-1-piperazinyl]carbonyl]pentyl]amino]-1-[(3,5-dibromo-4-hydroxyphenyl)methyl]-2-oxoethyl]-4-(1,4-dihydro-2-oxo-3(2H)-quinazoliny 1) (BIBN4096BS) affinity for calcitonin gene-related peptide and amylin receptors--the role of receptor activity modifying protein 1. Mol Pharmacol 2006; 70(6):1984-91.
Hay DL, Christopoulos G, Christopoulos A, Sexton PM. Amylin receptors: molecular composition and pharmacology. Biochem Soc Trans 2004; 32(Pt 5):865-7.
Hay DL, Christopoulos G, Christopoulos A, Poyner DR, Sexton PM. Pharmacological discrimination of calcitonin receptor: receptor activity-modifying protein complexes. Mol Pharmacol 2005; 67(5):1655-65.
Heilker R. High content screening to monitor G protein-coupled receptor internalisation. Ernst Schering Found Symp Proc 2006;(2):229-47.
Heilker R, Zemanova L, Valler MJ, Nienhaus GU. Confocal fluorescence microscopy for high-throughput screening of G-protein coupled receptors. Curr Med Chem 2005; 12(22):2551-9.
Jacoby E, Bouhelal R, Gerspacher M, Seuwen K. The 7 TM G-protein-coupled receptor target family. ChemMedChem 2006; 1(8):761-82.
Jonderko K. Gut. 1989 Apr;30(4):430-5.
Kostenis E. G proteins in drug screening: from analysis of receptor-G protein specificity to manipulation of GPCR-mediated signalling pathways. Curr Pharm Des 2006; 12(14):1703-15.
Kuestner RE, Elrod RD, Grant FJ, Hagen FS, Kuijper JL, Matthewes SL et al. Cloning and characterization of an abundant subtype of the human calcitonin receptor. Mol Pharmacol 1994; 46(2):246-55.
Lee SK, Goldring SR, Lorenzo JA. Expression of the calcitonin receptor in bone marrow cell cultures and in bone: a specific marker of the differentiated osteoclast that is regulated by calcitonin. Endocrinology 1995; 136(10):4572-81.
Leifert WR, Aloia AL, Bucco O, Glatz RV, McMurchie EJ. G-protein-coupled receptors in drug discovery: nanosizing using cell-free technologies and molecular biology approaches. J Biomol Screen 2005; 10(8):765-79.
Lin HY, Harris TL, Flannery MS, Aruffo A, Kaji EH, Gorn A et al. Expression cloning of an adenylate cyclase-coupled calcitonin receptor. Science 1991; 254(5034):1022-4.
Mangiafico C., Firoe C.E., Foti R., Lunetta M., Fargetta C., Grimaldi D.R. and Petralito A., La Riforma Medica, Vol. 103, No 12, 563-566.
Mansoor S, Youn YS, Lee KC. Pharm Dev Technol. 2005;10(3):389-96.
Nishikawa T, Ishikawa H, Yamamoto S, Koshihara Y. A novel calcitonin receptor gene in human osteoclasts from normal bone marrow. FEBS Lett 1999; 458(3):409-14.
Notoya et al; European Journal of Pharmacology, 560, 2007, 234-239
Nussenzveig DR, Mathew S, Gershengorn MC. Alternative splicing of a 48-nucleotide exon generates two isoforms of the human calcitonin receptor. Endocrinology 1995; 136(5):2047-51.
Oh DY, Kim K, Kwon HB, Seong JY. Cellular and molecular biology of orphan G protein-coupled receptors. Int Rev Cytol 2006; 252:163-218.
Passariello N, Giugliano D, Sgambato S, Torella R, D'Onofrio F. J Clin Endocrinol Metab. 1981 Aug;53(2):318-23. Calcitonin, a diabetogenic hormone?
Petralito A, Lunetta M, Liuzzo A, Fiore CE, Heynen G. J Endocrinol Invest. 1979 Apr-Jun;2(2):209-11. Effects of salmon calcitonin on blood glucose and insulin levels under basal conditions and after intravenous glucose load.
Pittner RA., Eur J Pharmacol. 1997 May 1;325(2-3):189-97.
Poyner DR, Sexton PM, Marshall I, Smith DM, Quirion R, Born W et al. International Union of Pharmacology. XXXII. The mammalian calcitonin gene-related peptides, adrenomedullin, amylin, and calcitonin receptors. Pharmacol Rev 2002; 54(2):233-46.
Prego C, Fabre M, Torres D, Alonso MJ Pharm Res. 2006 Mar;23(3):549-56. Epub 2006 Mar 16.
Prego C, Torres D, Alonso MJ. J Nanosci Nanotechnol. 2006 Sep-Oct;6(9-10):2921-8.
Prego C, Garcia M, Torres D, Alonso MJ. J Control Release. 2005 Jan 3;101(1-3):151-62.
Prego C, Torres D, Fernandez-Megia E, Novoa-Carballal R, Quiñoá E, Alonso MJ. J Control Release. 2006 Apr 10;111(3):299-308. Epub 2006 Feb 14.
Purdue BW, Tilakaratne N, Sexton PM. Molecular pharmacology of the calcitonin receptor. Receptors Channels 2002; 8(3-4):243-55.
Qi T, Christopoulos G, Bailey RJ, Christopoulos A, Sexton PM, Hay DL. Identification of N-terminal receptor activity-modifying protein residues important for calcitonin gene-related peptide, adrenomedullin, and amylin receptor function. Mol Pharmacol 2008; 74(4):1059-71.
Shen Z, Mitragotri S, Pharm Res. 2002 Apr;19(4):391-5 'Intestinal patches for oral drug delivery'.
Sinko PJ, Lee YH, Makhey V, Leesman GD, Sutyak JP, Yu H, Perry B, Smith CL, Hu P, Wagner EJ, Falzone LM, McWhorter LT, Gilligan JP, Stern W. Pharm Res. 1999 Apr;16(4):527-33.
Smith DM, Coppock HA, Withers DJ, Owji AA, Hay DL, Choksi TP et al. Adrenomedullin: receptor and signal transduction. Biochem Soc Trans 2002; 30(4):432-7.
Song KH, Chung SJ, Shim CK J Control Release. 2005 Sep 2;106(3):298-308.
Starke A, Keck E, Berger M, Zimmermann H. Diabetologia. 1981 May;20(5):547-52.
Takahashi S, Goldring S, Katz M, Hilsenbeck S, Williams R, Roodman GD. Downregulation of calcitonin receptor mRNA expression by calcitonin during human osteoclast-like cell differentiation. J Clin Invest 1995; 95(1):167-71.
Thomsen W, Frazer J, Unett D. Functional assays for screening GPCR targets. Curr Opin Biotechnol 2005; 16(6):655-65.
Waller A, Simons PC, Biggs SM, Edwards BS, Prossnitz ER, Sklar LA. Techniques: GPCR assembly, pharmacology and screening by flow cytometry. Trends Pharmacol Sci 2004; 25(12):663-9.
Xiao SH, Reagan JD, Lee PH, Fu A, Schwandner R, Zhao X et al. High throughput screening for orphan and liganded GPCRs. Comb Chem High Throughput Screen 2008; 11(3):195-215.
Yamin M, Gorn AH, Flannery MR, Jenkins NA, Gilbert DJ, Copeland NG et al. Cloning and characterization of a mouse brain calcitonin receptor complementary deoxyribonucleic acid and mapping of the calcitonin receptor gene. Endocrinology 1994; 135(6):2635-43.
Young AA, Wang MW, Gedulin B, Rink TJ, Pittner R, Beaumont K. Metabolism. 1995 Dec;44(12):1581-9. Diabetogenic effects of salmon calcitonin are attributable to amylin-like activity.
Young A; Advances in Pharmacology, Volume 52, 2005, pp. 193-207.
Ziegler R, Bellwinkel S, Schmidtchen D, Minne H. Horm Metab Res. 1972 Jan;4(1):60. Effects of hypercalcemia, hypercalcemia and calcitonin on glucose stimulated insulin secretion in man.
Zofková I, Nedvídková J, Stárka L, Zamrazil V. Exp Clin Endocrinol. 1987 Mar;89(1):91-6.
Zofková I, Zamrazil V. Horm Metab Res. 1987 Dec;19(12):656-60.
Zolnierowicz S, Cron P, Solinas-Toldo S, Fries R, Lin HY, Hemmings BA. Isolation, characterization, and chromosomal localization of the porcine calcitonin receptor gene. Identification of two variants of the receptor generated by alternative splicing. J Biol Chem 1994; 269(30):19530-8.

## Claims

1. A pharmaceutical formulation for enteral administration for treating type I diabetes, type II diabetes, or metabolic syndrome, or for mitigating insulin resistance, or for reducing an undesirably high fasting serum glucose level, or for reducing an undesirably high peak serum glucose level, or for reducing an undesirably high peak serum insulin level, which formulation comprises an active compound which is a calcitonin family member other than amylin, a modified calcitonin family member other than a modified amylin, or a calcitonin receptor agonist.

2. A formulation as claimed in claim 1, formulated for oral administration to the digestive tract.

3. A formulation as claimed in claim 1 or claim 2, wherein the active compound is a calcitonin.

4. A formulation as claimed in claim 3, wherein the calcitonin is salmon calcitonin.

5. A formulation as claimed in claim 1 or claim 2, wherein the active compound is of the general formula:
CX¹X²LSTCX³LX⁴X⁵X⁶X⁷X⁸X⁹X¹⁰X¹¹X¹²X¹³X¹⁴X¹⁵X¹⁶X¹⁷X¹⁸X¹⁹X²⁰X²¹GX²²X²³X²⁴XP
SEQ ID NO: 11
wherein:
X¹ is A, G, or S;
X² is N or S;
X³ is M or V;
X⁴ is G or S;
X⁵ is T, K, or A;
X⁶ is L or Y;
X⁷ is T, S, or W;
X⁸ is Q, K, or R;
X⁹ is D, E, or N;
X¹⁰ is F or L;
X¹¹ is N or H;
X¹² is K or N;
X¹³ is F or L;
X¹⁴ is H or Q;
X¹⁵ is T or R;
X¹⁶ is F or Y;
X¹⁷ is P or S;
X¹⁸ is Q, G or R;
X¹⁹ is T, I or M;
X²⁰ is A, N, D, S, or G;
X²¹ is I, T, V or F;
X²² is V, S, A, or P;
X²³ is G or E;
X²⁴ is A or T.

6. A formulation as claimed in claim 5, wherein the active compound is of the formula:
CSNLSTCVLGX⁵LX⁷QX⁹LHKLQTYPX¹⁸TNTGX²²GTP
SEQ ID NO:12
wherein
X⁵ is T or K;
X⁷ is T or S;
X⁹ is D or E;
X¹⁸ is Q or R;
X²² is V, S, or A.

7. A formulation as claimed in claim 1 or claim 2, wherein the active compound is a modified calcitonin family member having at least 75% amino acid homology with a calcitonin member other than amylin and being modified with respect to said calcitonin family member by addition, substitution or deletion of amino acids and retaining the ability to bind and to activate the calcitonin receptor.

8. A formulation as claimed in any preceding claim, wherein said active compound is formulated with a carrier for oral administration.

9. A formulation as claimed in claim 8, wherein the carrier increases the oral bioavailability of the active compound.

10. A formulation as claimed in claim 8, wherein the carrier comprises 5-CNAC, SNAD, or SNAC.

11. A method of treatment of type I diabetes, Type II diabetes, or metabolic syndrome, or for mitigating insulin resistance, or for reducing an undesirably high fasting serum glucose level, or for reducing an undesirably high peak serum glucose level, or for reducing an undesirably high peak serum insulin level, comprising enteral administration to a patient in need thereof for treatment of a said condition of a pharmaceutically effective amount of pharmaceutical formulation comprising an active compound which is a calcitonin family member other than amylin, a modified calcitonin family member other than a modified amylin, or a calcitonin receptor agonist, and optionally a carrier serving to enable effective enteral administration of said active compound.
